# EUROPEAN PATENT SPECIFICATION

(11) **EP 4 099 949 B1**
(45) Date of publication and mention of the grant of the patent: **18.11.2026**
(21) Application number: 21750707.8
(22) Date of filing: 05.02.2021
(51) Int. Cl.: A61F 2/16

(54) **ACCOMMODATING INTRAOCULAR LENSES AND ASSOCIATED METHODS**
AKKOMMODIERENDE INTRAOKULARLINSEN UND ZUGEHÖRIGE VERFAHREN
LENTILLES INTRAOCULAIRES ADAPTATIVES ET MÉTHODES ASSOCIÉES

(30) Priority: 05.02.2020 US 202062970612 P; 12.06.2020 US 202063038624 P
(43) Date of publication of application: 14.12.2022
(73) Proprietor: Shifamed Holdings, LLC, Campbell, CA 95008 (US)
(72) Inventor: SCHOLL, John, Campbell, California 95008 (US); PEREA, Juan Diego, Campbell, California 95008 (US); CROWLEY, Cornelius Matthew, Campbell, California 95008 (US); SAUL, Tom, Campbell, California 95008 (US); ZAHER, Amir Omar, Campbell, California 95008 (US); RAYBOULD, Owen, Campbell, California 95008 (US); LOGAN II, Benjamin Arthur, Campbell, California 95008 (US)
(74) Representative: Forresters IP LLP
(86) International application number: PCT/US2021/016760
(87) International publication number: WO 2021/158882

(56) References cited:
- WO-A1-2015/066532
- WO-A1-2016/018932
- US-A1- 2015 173 892
- US-A1- 2016 030 161
- US-A1- 2018 177 589
- US-A1- 2018 177 589
- US-A1- 2019 290 422
- US-B2- 10 159 564

## Description

### CROSS-REFERENCE TO RELATED APPLICATION(S)

This application claims priority to U.S. Provisional Patent Application No. 62/970,612, titled ACCOMMODATING INTRAOCULAR LENSES AND ASSOCIATED METHODS, filed February 5, 2020, and U.S. Provisional Patent Application No. 63/038,624, titled ACCOMMODATING INTRAOCULAR LENSES AND ASSOCIATED METHODS, filed June 12.

### TECHNICAL FIELD

The present technology relates to accommodating intraocular lenses (AIOLs) and methods of implanting and assembling the same.

### BACKGROUND

Cataracts can affect a large percentage of the worldwide adult population with clouding of the native crystalline lens and resulting loss of vision. Patients with cataracts can be treated by native lens removal and surgical implantation of a synthetic intraocular lens (IOL).

Worldwide, there are millions of IOL implantation procedures performed annually. In the U.S., there are 3.5 million cataract procedures performed, while worldwide there are over 20 million annual procedures performed.

Although IOL implantation procedures can be effective at restoring vision, conventional IOLs have several drawbacks. For example, many prior IOLs are not able to change focus as a natural lens would (known as accommodation). Other drawbacks of conventional IOLs include refractive errors that occur after implantation and require glasses for correcting distance vision, or in other cases the IOLs can be effective in providing good far vision, but patients need glasses for intermediate and near vision.

Several multi-focal IOLs have been developed to address these drawbacks, but they too can have drawbacks. For example, although multi-focal IOLs generally perform well for reading and distance vision, in at least some instances such multi-focal IOLs may cause significant glare, halos, reduced contrast sensitivity, and other visual artifacts.

AIOLs have been proposed to provide accommodative optical power in response to the distance at which a patient views an object. However, such AIOLs are generally still in development and have different drawbacks. For example, prior AIOLs can provide insufficient accommodation after implantation or produce suboptimal refractive correction of the eye. The amount of accommodation of the prior AIOLs can also decrease after implantation in at least some instances. The prior AIOLs can also be too large to be inserted through a small incision of the eye and may require the incision to be somewhat larger than would be ideal. Also, at least some of the prior AIOLs can be unstable when placed in the eye, which can lead to incorrect accommodation and other errors.

US 2018/177589 A1 discloses an accommodating intraocular lens (AIOL) for implantation within a capsular bag of a patient's eye comprises first and second components coupled together to define an inner fluid chamber and an outer fluid reservoir. In particular this document also discloses an optical structure with first and second optical components aligned along an optical axis; protrusions/posts/standoffs defining channels between reservoir and chamber; and a fixed lens detachably coupled to the accommodating structure.

US10159564 B2 discloses a two-part accommodating intraocular lens (IOL) device for implantation in a capsular bag of a patient's eye.
US 2016/030161 A1 discloses an accommodating intraocular lens (IOL) that can be implanted either alone or as part of a two-part lens assembly.

Improved implantable intraocular lenses that accommodate with the natural mechanisms of controlling focusing of the eye that overcome at least some of the above deficiencies would be desirable. Ideally, such improved AIOLs would provide increased amounts of accommodation when implanted, provide refractive stability, introduce few if any perceptible visual artifacts, and allow the optical power of the eye to change from far vision to near vision in response to the distance of the object viewed by the patient.

The present invention relates to an accommodating intraocular lens as set forth in the claims.

### BRIEF DESCRIPTION OF THE DRAWINGS

Many aspects of the present technology can be better understood with reference to the following drawings. The components in the drawings are not necessarily drawn to scale. Instead, emphasis is placed on illustrating clearly the principles of the present technology. Furthermore, components can be shown as transparent in certain views for clarity of illustration only and not to indicate that the component is necessarily transparent. Components may also be shown schematically.
FIG. 1A illustrates an anterior-posterior elevation view of an embodiment of an AIOL.
FIG. 1B illustrates the accommodating structure of the AIOL of FIG. 1A in an exploded configuration.
FIG. 1C illustrates a cross-sectional view of the AIOL of FIG. 1A along cut-plane A-A of FIG. 1A.
FIG. 1D illustrates a cross-sectional view of the AIOL of FIG. 1A along cut-plane B-B of FIG. 1A.
FIG. 2A illustrates a perspective view of an AIOL configured in accordance with an embodiment of the present technology.
FIG. 2B illustrates a cross-sectional view of the AIOL of FIG. 2A along a cut-plane parallel to and passing through the central optical axis of the AIOL of FIG. 2A.
FIG. 2C illustrates a perspective cross-sectional view of a first component of an accommodating structure of the AIOL of FIG. 2A along a cut-plane parallel to and passing through the central optical axis of the AIOL of FIG. 2A.
FIG. 2D illustrates a lower perspective view of the first component of FIG. 2C.
FIG. 2E illustrates a cross-sectional view of a portion of the accommodating structure of the AIOL of FIG. 2A along a cut-plane parallel to and passing through the central optical axis of the AIOL of FIG. 2A.
FIG. 2F illustrates a cross-sectional view of a portion of the accommodating structure of the AIOL of FIG. 2A along a cut-plane parallel to and passing through the central optical axis of the AIOL of FIG. 2A, with the addition of a groove in a posterior surface of the first portion of the accommodating structure of the AIOL of FIG. 2A.
FIG. 2G illustrates a lower perspective view of a first component of an AIOL configured in accordance with an embodiment of the present technology, wherein the ring portion includes standoffs extending in a direction parallel to the central optical axis of the first component.
FIG. 2H illustrates a cross-sectional view of a portion of an accommodating structure of an AIOL having the first component of FIG. 2G, taken along a cut-plane parallel to and passing through the central optical axis of the first component of FIG. 2G.
FIG. 3A illustrates a perspective view of an AIOL configured in accordance with another embodiment of the present technology.
FIG. 3B illustrates a cross-sectional view of the AIOL of FIG. 3A along a cut-plane parallel to and passing through the central optical axis of the AIOL of FIG. 3A.
FIG. 3C illustrates a cross-sectional view of a portion of the accommodating structure of the AIOL of FIG. 3A along a cut-plane parallel to and passing through the central optical axis of the AIOL of FIG. 3A.
FIG. 3D illustrates a cross-sectional view of a portion of the AIOL of FIG. 3A along a cut-plane parallel to and passing through the central optical axis of the AIOL of FIG. 3A, wherein the outer bellows is shortened.
FIG. 4 illustrates a perspective view of an AIOL configured in accordance with an embodiment of the present technology.
FIG. 5A illustrates a perspective view of a fixed lens configured in accordance with an embodiment of the present technology.
FIG. 5B illustrates a perspective view of an AIOL configured in accordance with an embodiment of the present technology having the fixed lens of FIG. 5A.
FIG. 5C illustrates a plan view of the anterior face of the AIOL of FIG. 5B.
FIG. 6A illustrates a perspective view of a fixed lens configured in accordance with an embodiment of the present technology.
FIG. 6B illustrates a perspective view of an AIOL configured in accordance with an embodiment of the present technology having the fixed lens of FIG. 6A.
FIG. 6C illustrates a plan view of the anterior face of the AIOL of FIG. 6B.
FIG. 7A illustrates a perspective view of a fixed lens configured in accordance with an embodiment of the present technology.
FIG. 7B illustrates a perspective view of an AIOL configured in accordance with an embodiment of the present technology having the fixed lens of FIG. 7A.
FIG. 7C illustrates a plan view of the anterior face of the AIOL of FIG. 7B.
FIG. 8 illustrates a plan view of the anterior face of an AIOL configured in accordance with an embodiment of the present technology.
FIG. 9A illustrates a perspective view of an AIOL configured in accordance with an embodiment of the present technology.
FIG. 9B illustrates a lateral plan view of a side of the AIOL of FIG. 9A.
FIG. 9C illustrates a plan view of the anterior face of the AIOL of FIG. 9A.
FIG. 9D illustrates a cross-sectional view of the AIOL of FIG. 9A, along a cut-plane 9-9 of FIG. 9C.
FIG. 10A illustrates an exploded perspective view of an AIOL configured in accordance with an embodiment of the present technology.
FIG. 10B illustrates a plan view of the anterior face of the AIOL of FIG. 10A.
FIG. 10C illustrates a lateral plan view of a side of the AIOL of FIG. 10A in a non-accommodating configuration.
FIG. 10D illustrates a lateral plan view of a side of the AIOL of FIG. 10A in an accommodating configuration.
FIG. 10E illustrates a cross-sectional view of the AIOL of FIG. 10A, along the cut-plane 10-10 of FIG. 10B and in a non-accommodating configuration.
FIG. 10F illustrates a cross-sectional view of the AIOL of FIG. 10A, along the cut-plane 10-10 of FIG. 10B and in an accommodating configuration.
FIG. 10G illustrates a close-up view of the cross-sectional view of FIG. 10E.
FIG. 10H illustrates a close-up view of the cross-sectional view of FIG. 10F.
FIG 11A illustrates an exploded perspective view of an AIOL configured in accordance with an embodiment of the present technology.
FIGS. 11B and 11C illustrate perspective views of the AIOL of FIG. 11A when the fixed lens is received in receiving structures of the accommodating structure.
FIG. 11D illustrates a cross-sectional view of the AIOL of FIG. 11C taken along the optical axis of the AIOL of FIG. 11A.
FIG. 11E illustrates a top cross-sectional view of the AIOL of FIG. 11A with bumps on an inner surface of the accommodating portion and with the fixed lens in a first position.
FIG. 11F illustrates the AIOL of FIG. 11E with the fixed lens in a second position.
FIG. 11G illustrates a top cross-sectional view of the AIOL of FIG. 11A with steps on an inner surface of the accommodating portion and with the fixed lens in a first position.
FIG. 11H illustrates the AIOL of FIG. 11G with the fixed lens in a second position.
FIG. 12A illustrates an exploded perspective view of an AIOL configured in accordance with an embodiment of the present technology.
FIG. 12B illustrates a perspective view of the AIOL of FIG. 12A.
FIG. 13A illustrates an exploded view of an AIOL configured in accordance with an embodiment of the present technology.
FIG. 13B illustrates the AIOL of FIG. 13A wherein a fixed lens of the AIOL is coupled to a base lens of the AIOL.
FIG. 13C illustrates a plan view of the anterior face of the AIOL of FIG. 13B.
FIG. 13D illustrates a lateral cross-sectional view of the AIOL of FIG. 13B, taken along the cut-plane 13D-13D of FIG. 13C.
FIG. 14A illustrates an exploded perspective view of an AIOL configured in accordance with an embodiment of the present technology.
FIG. 14B illustrates a plan view of the anterior face of the AIOL of FIG. 14A.
FIG. 15A illustrates a plan view of the anterior face of a second component of an accommodating structure of an AIOL configured in accordance with an embodiment of the present technology.
FIG. 15B illustrates a close-up perspective view of a filling portion of the second component of FIG. 15A.
FIG. 15C illustrates a close-up cross-sectional view of the filling portion of the second component of FIG. 15A with a needle extending therethrough.
FIG. 15D illustrates a close-up cross-sectional view of the filling portion of the second component of FIG. 15A with a needle extending therethrough at a non-zero angle with respect to the optical axis of the second component of FIG. 15A.

### DETAILED DESCRIPTION

The present technology is directed to AIOLs and methods for making and using such devices. In many of the embodiments disclosed herein, the AIOLs include an accommodating lens portion and a fixed lens portion configured to removably connect to the accommodating lens portion. The AIOLs can include indentations (e.g., crenels, channels, valleys, dimples, trenches, , etc.) and protrusions (e.g., merlons, ridges, bumps, etc.) on an anterior edge of the bellows of the AIOL to facilitate passage of fluid past the AIOL when the AIOL is implanted in a lens capsule of a patient. In some embodiments, the AIOL includes a haptic portion comprising a plurality of spring elements connected to each other by one or more frame components. In some embodiments, the AIOL includes a removable fixed lens and a removable accommodating lens.

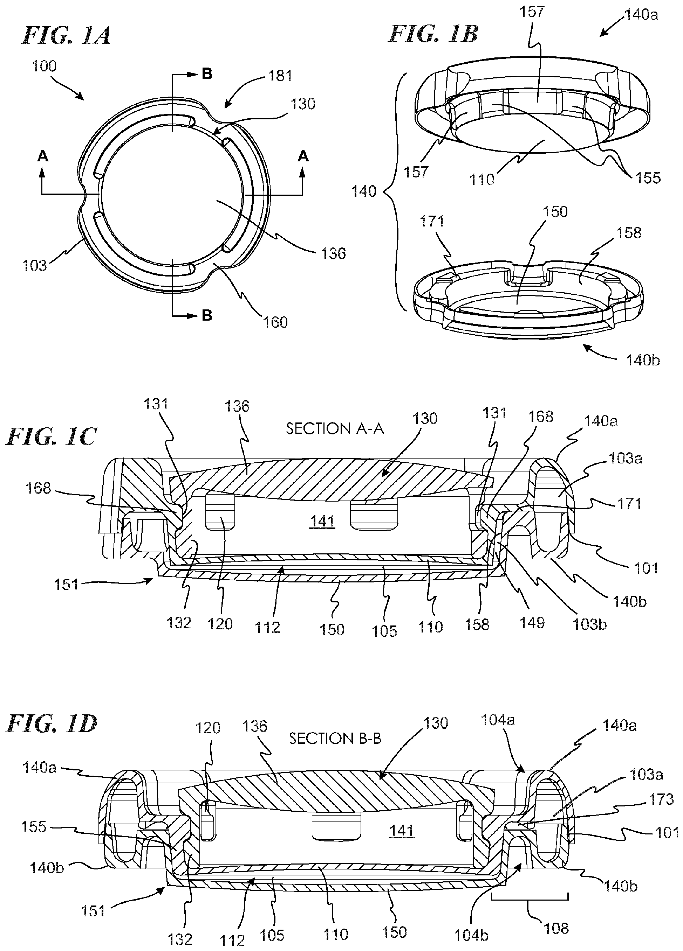

Specific details of various embodiments of the present technology are described below with reference to FIGS. 1A-10H. Although many of the embodiments are described below with respect to AIOLs and associated methods, other embodiments are within the scope of the present technology. Additionally, other embodiments of the present technology can have different configurations, components, and/or procedures than those described herein. For instance, AIOLs configured in accordance with the present technology may include additional elements and features beyond those described herein, or other embodiments may not include several of the elements and features shown and described herein.

For ease of reference, throughout this disclosure identical reference numbers are used to identify similar or analogous components or features, but the use of the same reference number does not imply that the parts should be construed to be identical. Indeed, in many examples described herein, the identically numbered parts are distinct in structure and/or function.

FIGS. 1A-1D illustrate an embodiment of an AIOL 100 including channels for fluid to flow from an outer fluid reservoir to an inner fluid chamber. Referring to FIGS. 1A and 1B together, the AIOL 100 includes an accommodating structure 140 (e.g., a base lens) having a first component 140a (e.g., an anterior lens component) and a second component 140b (e.g., a posterior lens component). In some embodiments, the first and second components 140a and 140b are assembled to form an outer fluid reservoir 103 (e.g., a haptic reservoir) (FIG. 1A), a mid-bellows channel 173 (FIG. 1D), and an inner fluid chamber 105 (e.g., an optical chamber) (FIGS. 1C-1D). The first component 140a of the accommodating structure 140 can have an inner portion with a first optical component 110, standoffs 155, and recesses 157 between the standoffs 155. The standoffs 155 project radially outward from the recesses 157. The second component 140b of the accommodating structure 140 can have an inner portion with a second optical component 150 and a wall 158. Referring to FIGS. 1C and 1D, which are cross-sectional views taken along lines A-A and B-B of FIG. 1A, respectively, the standoffs 155 contact the wall 158 (FIG. 1D) such that the recesses 157 (FIG. 1B) define channels for fluid to flow from the mid-bellows channel 173 to the fluid chamber 105.

As illustrated in FIG. 1D, the standoffs 155 project radially outward to engage the wall 158. The standoffs 155 of the AIOL 100 accordingly do not extend into the optical region of the AIOL, which increases the field of view of the AIOL 100.

Referring again to FIGS. 1B and 1C, one or both of the first and second components 140a, 140b of the accommodating structure 140 can include axial projections, standoffs, spacers, protrusions, or other features configured to space a portion of the first component 140a from the second component 140b in a direction parallel to an optical axis of the AIOL 100. For example, the second component 140b can include one or more projections 171 extending from an anterior edge of the wall 158 toward the first component 140a. Space between the projections 171 can form the mid-bellows channels 173 described below. In some embodiments, one or both of the first and second components 140a, 140b include indentations that define, at least in part, the mid-bellows channels 173. Such indentations can be included in addition to or instead of the projections 171. For example, the anterior edge of the wall 158 can include one or more indentations and/or channels.

In some embodiments, the AIOL 100 includes flow-through features 181 that enhance the rate and ease with which Ophthalmic Viscosurgical Devices (OVDs) used during the implantation of AIOLs can be removed from the natural lens capsule. The embodiment of the AIOL 100 illustrated in FIGS. 1A-1D comprises three outer flow-through features 181. The outer flow-through features 181 can be detents, such as a recess, distributed circumferentially along the perimeter of the outer fluid reservoir 103. The flow-through features 181 can create passages between the outer perimeter of the AIOL 100 and an inner surface of an eye capsule in which the AIOL 100 is implanted to allow fluid flow around an outer perimeter of the AIOL 100. In the illustrated embodiment, the flow-through features 181 are formed in regions of the first and second components 140a and 140b. Although three outer flow-through features 181 are illustrated, other embodiments may comprise fewer or more than illustrated. The outer flow-through features 181 may additionally provide rotational constraint to maintain the rotational orientation of the accommodating structure 140 with respect to a patient's eye capsule when implanted.

The embodiment of the AIOL 100 additionally comprises a fixed lens assembly 130. The fixed lens assembly 130 illustrated in FIGS. 1C-D includes an optical portion 136, a skirt 132 extending from the optical portion 136, and passages 120. The optical portion 136 can have a fixed power which may comprise an asymmetrically powered lens (e.g., a toric lens) or other lens, and the passages 120 are holes, slots, orifices, etc., that pass through the skirt 132 and extend into a perimeter region but not the optical portion 136.

Referring to FIG. 1C, the fixed lens assembly 130 can have an engagement feature 131, such as an annular groove, that extends around the skirt 132, and the first component 140a of the accommodating structure 140 can have a thickened region 168, such as an annular protrusion (e.g., a ledge) that extends radially inwardly. The fixed lens assembly 130 can be releasably attached to the accommodating structure 140 by engaging the continuous thickened region 168 of the first component 140a with the engagement feature 131 of the fixed lens 130. In other embodiments (not shown), the thickened region 168 and the engagement feature 131 may be discontinuous features (e.g., segmented or other recesses or protrusions that extend around less than the full circumference of the fixed lens assembly 130 and the accommodating structure 140). Such a discontinuous thickened region 168 and engagement feature 131 can facilitate maintenance of a particular radial alignment between the fixed lens assembly 130 and the accommodating structure 140, such as when the fixed lens 130 comprises a toric lens or other asymmetrical lens. Alternatively, the groove may be in the fixed lens 130 and the protrusion on the accommodating structure 140.

The AIOL 100 can have a fluid accommodating lens 112 defined by a fluid chamber 105 (FIGS. 1C and 1D) bounded between a first optical component 110 and a second optical component 150. The fluid chamber 105 is in fluid communication with the outer reservoir 103 via discrete fluid channels 149 between standoffs 155 when the first and second components 140a and 140b are assembled. The first and second optical components 110 and 150 may be planar members (e.g., optical membranes) of the first and second components 140a and 140b, respectively. The first and second optical components 110 and 150, for example, can be integrally formed as optical membranes with the other portions of the first and second components 140a and 140b. In alternate embodiments, either or both of the membranes of the first and second optical components 110 and 150 may be a lens (i.e., have an optical power).

The AIOL 100 can further include a square-shaped (e.g., stepped) annular region 151 that inhibits cell migration from the periphery of the patient's capsule to the optical part of AIOL 100 (shown in FIGS. 1C-D at the posterior most region of the lens). Inhibiting cell migration from the periphery of the patient's capsule to the optical part of the AIOL 100 can reduce the risk of post-surgery opacification of the optical system.

The peripheral portions of the first component 140a and the second component 140b define the outer fluid reservoir 103, and the inner portions of the first and second components 140a and 140b define the accommodating structural element 140. The first and second components 140a and 140b can be bonded together at a seam 101. Means of bonding are described in detail in PCT Pub. No. WO2018/119408, appended to the end of the present disclosure. The first and second components 140a and 140b can also be bonded at other areas, such as at the standoffs 155. The standoffs 155 are separated by spaces that define fluid channels between the outer fluid reservoir 103 and the inner fluid chamber 105. The outer fluid reservoir 103 can be a bellows 108 having an outer bellows region 103a and an inner bellows region 103b, and the inner bellows region 103b can be defined by the channels between the standoffs 155.

In some embodiments, the volume of the inner bellows region 103b is less than the outer bellows region 103a. By reducing the volume of the inner bellows region 103b, additional space surrounding the optical region of the AIOL allows the optical aperture of the fixed lens 130 to be larger compared to embodiments with larger inner bellows regions. Additionally, the passages 120 of the fixed lens 130, which allow aqueous fluid to freely flow in and out of the chamber 141, are configured to pass through the outer skirt 132 and, in some embodiments, not the top optical portion 136. This is expected to reduce unwanted scattered light from internal reflections which may pass through the optical system and reach the retina.

The first component 140a may also comprise one or more thickened regions 160 for use, for instance, in filling the AIOL with an optical fluid. The thickened region 160 allows for a longer path for a needle used to fill the accommodating structure with optical fluid while a second needle in a different region is used to remove the gases the fluid is replacing. As illustrated, the fluid fill thickened region 160 is located adjacent one or more of the outer fluid flow-throughs 181. In some embodiments, the optical fluid may be comprised of a high refractive index poly vinyl alcohol.

Referring to FIG. 1D, the outer fluid reservoir 103 of the AIOL 100 can comprise (a) a first bellows structure 103a with an anterior portion 104a and a posterior portion 104b, (b) a second bellows structure 103b radially inward of the first bellows structure 103a, and/or (c) the mid-bellows channel 173 defining a horizontal passageway between the first and second bellows structures 103a and 103b. During operation as the capsule contracts, a mid-portion of the first bellows structure 103a can be constrained by the mid-bellows channel 173 while the anterior and posterior portions 104a and 104b of the first bellows structure 103a move radially inward with respect to the mid-bellows channel 173. The anterior and posterior portions 104a and 104b of the first bellows structure 103a can accordingly flex radially inward in response to the same amount of movement of the native capsule. This can cause more fluid to flow from the outer fluid reservoir 103 to the inner fluid chamber 105 and thereby provides more accommodation because anterior-posterior collapse of the outer fluid reservoir 103 is less efficient than radial compression of the outer fluid reservoir 103. Embodiments such as, but not limited to, any of those illustrated herein may be constructed from parts in which some or all of the portions not in the optical path have been dyed or treated to reduce light throughout to limit the ability of stray light entering portions outside the optical path from scattering into the optical path.

The fixed lens described in any of the embodiments described herein may be of spherical, aspheric, toric, or any other known lens configuration. Alternatively, or in combination, the fixed solid lens may be plano-convex, convex-concave, or convex-convex. The fixed lens may be configured to have positive or have negative fixed power.

The fluid lenses described herein may be configured such as to have one or more accommodating surfaces (e.g., two accommodating surfaces).

In some embodiments, instead of membranes without a power, the accommodating structure can include one or more deformable lenses that deflect based upon fluid pressure within the inner fluid chamber. The deformable lenses can each or both have a fixed power that can be positive or negative.

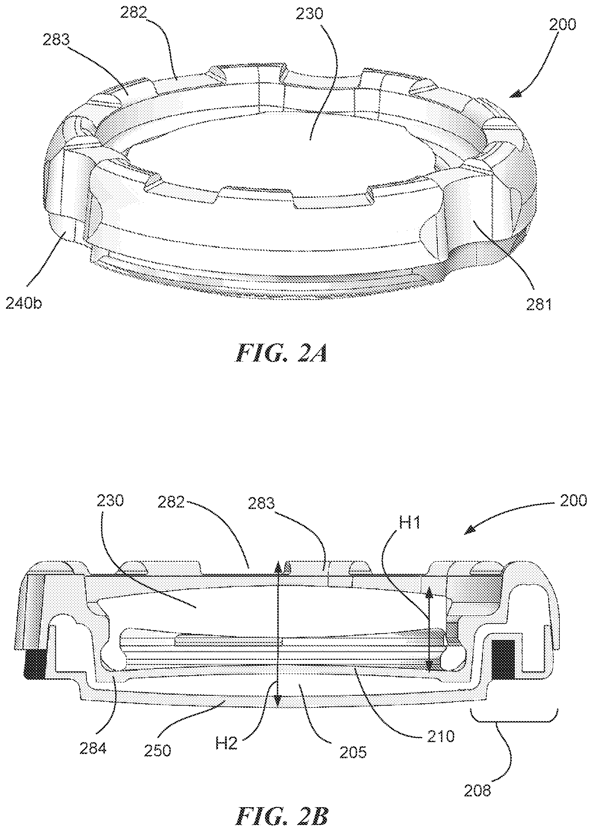

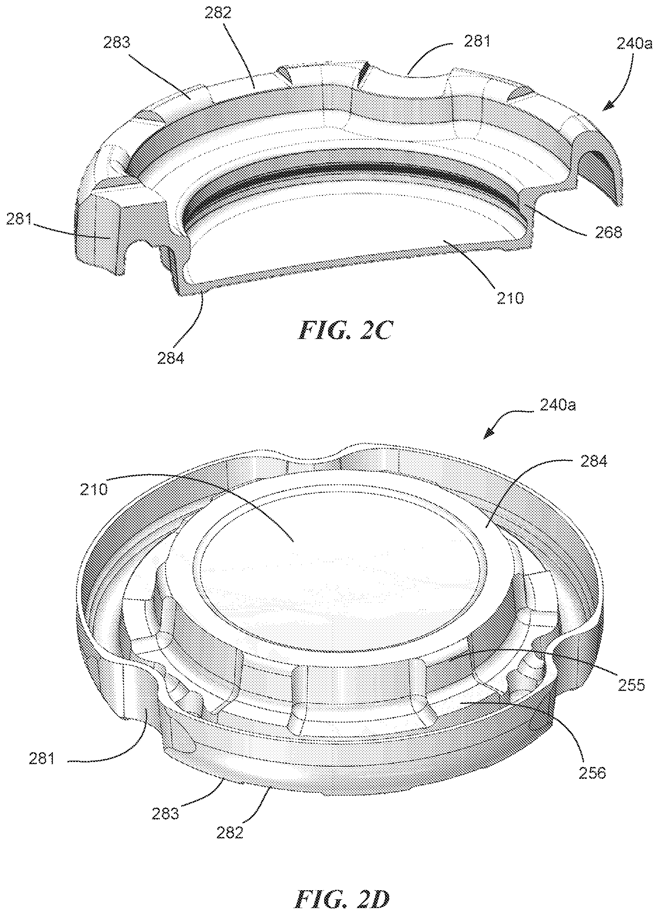

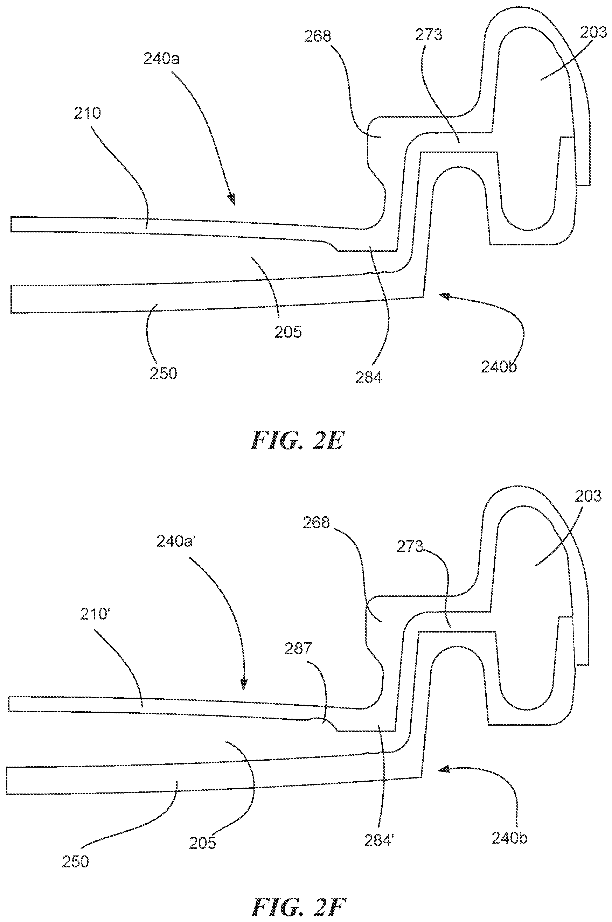

FIGS. 2A-2E illustrate an AIOL 200 having many or all of the same features of AIOL 100 described above with respect to FIGS. 1A-1D. For example, like reference numbers between FIGS. 2A-2E and FIGS. 1A-1D indicate identical or similar features (e.g., fixed lens 230 v. fixed lens 130). As illustrated, the AIOL 200 can include one or more indentations 282. The indentations 282 can be on an anterior edge of the first component 240a of the accommodating structure 240. Protrusions 283 can be formed between the indentations 282. Inclusion of indentations 282, protrusions 283, and/or flow-through features 281 is expected to improve fluid flow and aqueous circulation within the lens capsule past the anterior edge of the accommodating structure 240 when the AIOL 200 is implanted within the capsule of the patient. For example, the protrusions 283 can contact an anterior portion and/or lateral portion of the capsule while the indentations 282 form fluid pathways past the protrusions 283. In some embodiments, the protrusions 283 are sized such that the contact area between each protrusion 283 and the capsule is minimized. For example, one or more of the protrusions 283 can be sized such that maximum distance between a centroid of the contact area and the edges of the contact area is less than 300% of the thickness of the capsule membrane, less than 250% of the thickness of the capsule membrane, less than 200% of the thickness of the capsule membrane, less than 150% of the thickness of the capsule membrane, and/or less than 110% of the thickness of the capsule membrane. In some embodiments, the maximum distance between a centroid of the contact area and the edges of the contact area is approximately 100% of the thickness of the capsule membrane. Reducing the size of the contact areas between the protrusions 283 and the capsule can encourage/improve uniform diffusion of fluid through the membrane of the capsule at and near the contact areas.

The indentations 282 can have an arcuate width between 5°-30°, between 10°-15°, between 20°-25°, between 15°- 20°, and/or between 10°-20°. In some embodiments, the indentations 282 arcuate width is less than 30°, less than 25°, less than 20°, less than 15°, and 10°, and/or less than 5°. In some embodiments, each of the indentations 282 have approximately the equivalent arcuate width. One or more indentations 282 may have a greater arcuate width than one or more other indentations 282. Similarly, the protrusions 283 can have an arcuate width between 5°-30°, between 10°-15°, between 20°-25°, between 15°- 20°, and/or between 10°-20°. In some embodiments, the arcuate width of the protrusions 283 is less than 30°, less than 25°, less than 20°, less than 15°, and 10°, and/or less than 5°. In some embodiments, each of the protrusions 283 have approximately the equivalent arcuate width. One or more protrusions 283 may have a greater arcuate width than one or more other protrusions 283. These arcuate widths are measured with respect to a central optical axis of the accommodating structure 240. In some embodiments, one or more protrusions 283 have an arcuate width less than or greater than an arcuate width of one or more of the indentations 282. Further, one or more protrusions 283 may have an arcuate width equal to an arcuate width of one or more of the indentations 282.

As illustrated in FIGS. 2B-2E, the first component 240a of the accommodating structure 240 can include a ring portion and/or stiffening portion 284. The stiffening portion 284 can extend around the perimeter of the first optical component 210 of the first component 240a. The stiffening portion 284 can be, for example, a thickened portion of the first optical component 210. In some embodiments, the stiffening portion 284 is configured to reduce the likelihood of buckling and/or other undesired deformation of the first optical component (that may induce aberrations in the optical wave front passing through the AIOL) when compressive forces are applied to the bellows 208 (FIG. 2B). For example, the stiffening portion 284 can reduce the likelihood that the first optical component 210 deflects or deforms unevenly about the perimeter of the first optical component 210. In some embodiments, the stiffening portion 284 has a thickness (as measured parallel to the central optical axis of the accommodating structure 240) between 110%-150%, between 115%-200%, between 120%-140%, and/or between 125%-145% of the thickness of the first optical component 210 (as measured parallel to and along the central optical axis of the accommodating structure 240). In some embodiments, the stiffening portion 284 has a thickness of approximately 125% of the thickness of the first optical component 210. In some embodiments, the stiffening portion 284 has a radial thickness (as measured perpendicular to the central optical axis of the accommodating structure 240) between 5%-40%, between 10%-35%, between 10%-20%, and/or between 15%-30% of the radius of the first optical component 210 (as measured perpendicular to the central optical axis of the accommodating structure 240). In some embodiments, stiffening portion 284 has a radial thickness (e.g., width) of approximately 15% of the radius of the first optical component 210. The stiffening portion 284 can be formed as a monolithic part with the first optical component 210, or as a separate part later coupled with the first optical component 210.

As illustrated in FIG. 2B, the second optical component 250 can be thicker than the first optical component 210, as measured parallel to the central optical axis of the accommodating structure 240. Thickening the second optical component 250 is expected to reduce undesired/non-uniform deformation of the second optical component 250 when radially-compressive force is applied to the bellows 208. In some embodiments, for example, the second optical component 250 is up to 5% thicker, up to 10% thicker, up to 15% thicker, up to 20% thicker, up to 30% thicker, and/or up to 50% thicker than the first optical component 210.

In some embodiments, the first component 240a of the accommodating structure 240 includes a plurality of standoffs 255 similar to or the same as the standoffs 155 described above with respect to the AIOL 100. The standoffs 255 can extend in a generally radially-outward direction with respect to the central optical axis of the accommodating structure 240. In some embodiments, the radially outward surfaces of the standoffs 255 can be positioned any distance from the central optical axis of between 95%-98% of the distance between the central optical axis and the surface of the second component 240b to which the standoffs 255 are connected during manufacture. In some embodiments, the radially outward surfaces of the standoffs 255 are positioned at approximately the same radial distance from central optical axis as the surface of the second component 240b to which the standoffs 255 are connected during manufacturing. Sizing the standoffs 255 to approximately 100% the radial distance to the connecting surface of the second component 240b can reduce or eliminate mechanical stresses imparted on the first optical component 210 from the second component 240b of the accommodating structure 240.

In some embodiments, as illustrated in FIG. 2D, the first component 240a includes axial standoffs 256. The axial standoffs 256 can be connected to the standoffs 255 or separate from the standoffs 255. In the illustrated embodiment, for example, the axial standoffs 256 are connected to the standoffs 255 and project in a direction parallel to the central optical axis of the accommodating structure 240. In some embodiments, the axial standoffs 256 have an arcuate width greater than or less than the arcuate width of the standoffs 255. In some embodiments, the axial standoffs 256 have an arcuate width approximately equal to the arcuate width of the standoffs 255. Including axial standoffs 256 in addition to the standoffs 255 can increase stiffness and rigidity of the bond between the first component 240a of the accommodating structure 240 and the second component 240b of the accommodating structure 240. Increasing the rigidity of the bond between the first and second components 240a, 240b is expected to reduce the likelihood of undesired deformation of one or both of the first optical component 210 and the second optical component 250 in response to radial compression of the bellows 208.

FIG. 2F illustrates an embodiment of a first component 240a' generally similar to or identical to the first component 240a described above with respect to FIGS. 2A-2E. The first portion 240a' differs from the first component 240a described above in that the first portion 240a' includes one or more channels or grooves formed (e.g., cut, molded, or otherwise created) in the first optical component 210'. For example, a groove 287 can be formed on the posterior surface of the first optical component 210'. The groove 287 can be annular. In some embodiments, two or more grooves are formed on the posterior surface of the first optical component 210'. The groove 287 can be positioned in contact with or near the stiffening portion 284'. In some embodiments, the groove 287 is positioned at or near a radially outward edge (as measured perpendicular to the central optical axis of the accommodating structure 240) of the first optical component 210'. In some embodiments, the groove 287 is formed on the anterior surface of the first optical component 210'. The groove 287 can create a weakened portion of the first optical component 210'. This weakened portion can create a hinge point about which the first optical component 210' can deflect when transitioning between accommodating and non-accommodating configurations. Creating a weakened portion spaced radially-inward (as measured perpendicular to the central optical axis of the accommodating structure 240) from the outer edge of the first optical component 210' can reduce the diameter of the portion of the first optical component 210' that flexes in response to ingress of fluid into the inner fluid chamber 205 from the outer fluid reservoir 203. Reducing the diameter of the flexing portion of the first optical component 210' can reduce the amount of fluid ingress required to realize a given optic change for the first optical component 210' (e.g., less fluid is required to move radial center of the first optical component 210' a given distance in a direction parallel to the central optical axis of the accommodating structure 240). In some embodiments, creating a weakened portion in the first optical component 210' can increase circumferential uniformity in the deflection or deformation of the first optical component 210'.

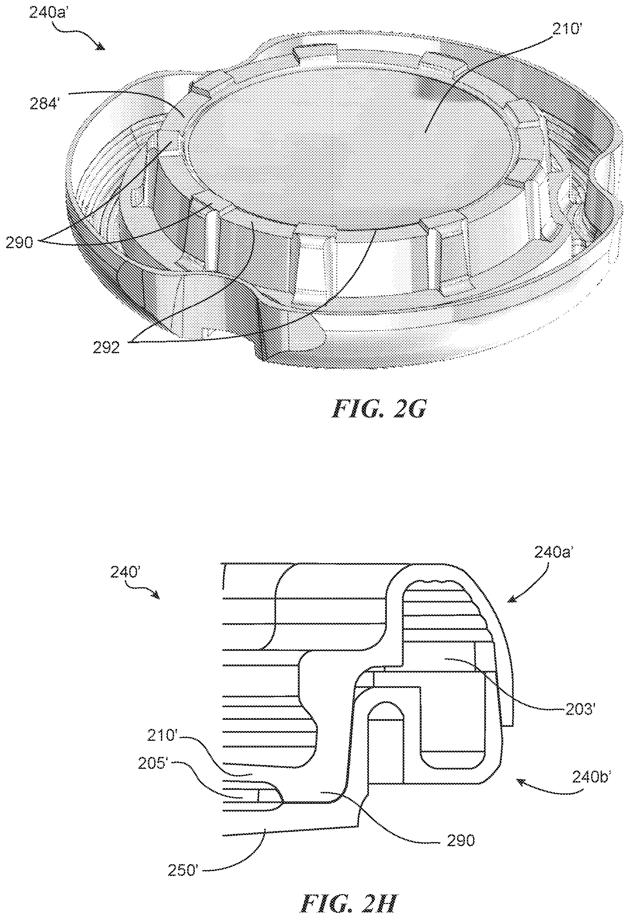

FIGS. 2G and 2H illustrate an AIOL 240' configured in accordance with an embodiment of the present technology. Elements having similar structural and/or functional characteristics as those described above with respect to FIGS. 2A-2F are designated by use of an apostrophe ('). For example, the first portion 240a' of the AIOL 240' is similar to the first portion 240a of the AIOL 240 described above. Referring to FIG. 2G, the first portion 240a' can include one or more standoffs 290 extending from a stiffening portion 284' in a direction parallel to the central optical axis of the first portion 240a'. As best seen in FIG. 2H, when the AIOL 240' is assembled, these standoffs 290 extend toward a second optical component 250' of a second component 240b' of the AIOL 240'. The standoffs 290 can contact the second optical component 250' (e.g., a perimeter portion thereof). Referring back to FIGS. 2G and 2H together, gaps 292 between standoffs 290 can allow fluid to pass from between an inner fluid chamber 205' of the AIOL 240' and an outer fluid reservoir 203' of the AIOL 240'. Use of the standoffs 290 as connecting points between the first and second components 240a', 240b' of the AIOL 240' can strengthen the bonding between the two components 240a', 240b' and reduce the risk of unintentional separation between the two components 240a', 240b' during storage, implantation, and/or operation. The standoffs can reduce the risk of aberrations (e.g., non-circular flexing during accommodation) of one or both of the first optical component 210' and the second optical component 250'.

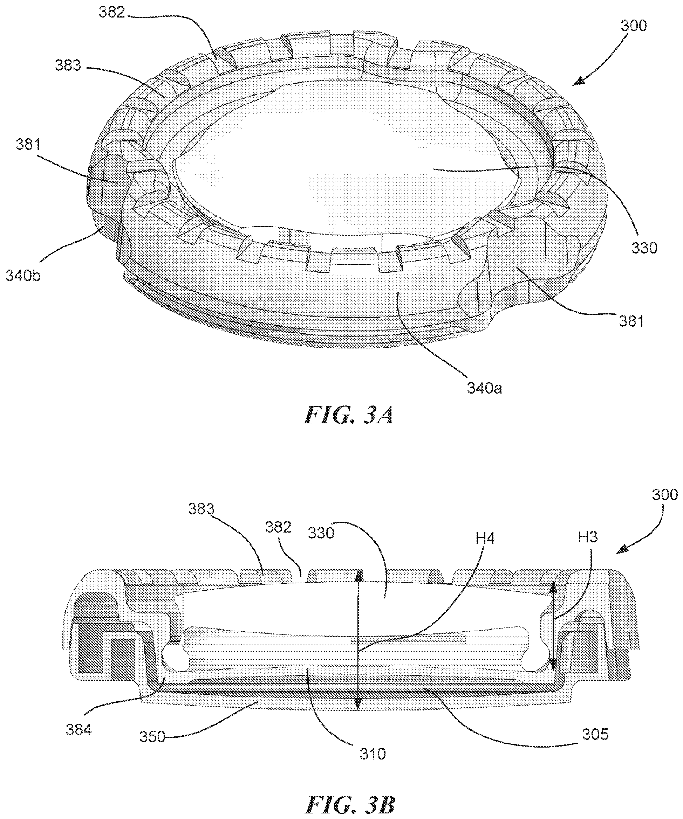

FIGS. 3A-3C illustrate an AIOL 300 having many or all of the same features of AIOLs 100, 200 described above with respect to FIGS. 1A-2E. For example, like reference numbers between FIGS. 3A-3C and FIGS. 1A-2E indicate identical or similar features (e.g., second optical component 150 v. second optical component 250 v. second optical component 350). AIOL 300 includes indentations 382 and/or protrusions 383 arranged about a periphery of the device. The indentations 382 and/or protrusions 383 of AIOL 300 have shorter arcuate width than the indentations 282 and/or protrusions 283 of AIOL 200 described above. In some embodiments, a fixed lens 330 of the AIOL 300 has a greater max height (as measured parallel to the central optical axis of the accommodating structure 340) as a proportion of the max height of the accommodating structure 340 than the fixed lens 230 with respect to the accommodating structure 240. For example, as illustrated in FIG. 2B, the fixed lens 230 can have a height H1 between 30%-60%, between 20%-70%, between 50%-60%, and/or between 40%-55% of the height H2 of the accommodating structure 340. In some embodiments, the fixed lens 230 can have a height H1 of approximately 55% of the height H2 of the accommodating structure 240. The fixed lens 330, as illustrated in FIG. 3B can have a height H3 between 35%-70%, between 45%-75%, between 40%-60%, and/or between 60%-65% of the height H4 of the accommodating structure 340. In some embodiments, the height H3 of the fixed lens 330 is approximately 62.5% of the height H4 of the accommodating structure 340. In some embodiments, reducing the height of the fixed lens 330 can allow for easier flushing of materials (e.g., OVDs or other materials) used during implantation of the AIOL 300.

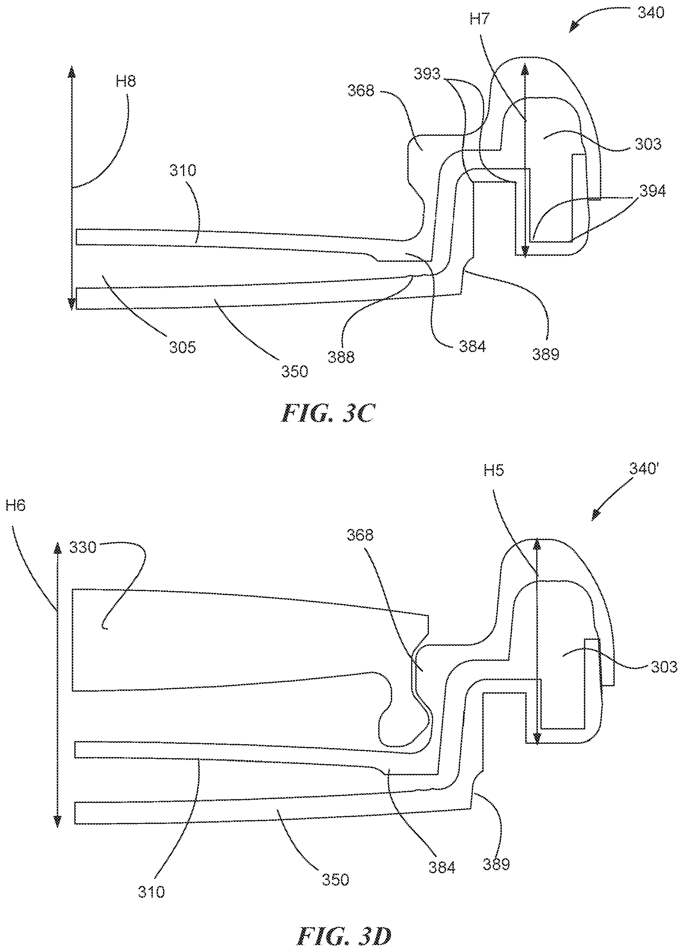

In some embodiments, as best seen in FIG. 3C, the second optical component 350 can include one or more ridges, undulations, or other surface features on the anterior surface of the second optical component 350. For example, one or more annular ridges 388 can protrude from the anterior surface of the second optical component 350. The ridges 388 can inhibit or prevent passage of surface treatments applied to the anterior surface of the second optical component 350 beyond the ridges 388. Inhibiting or preventing surface treatments from going beyond the ridges 388 can reduce the likelihood that the surface treatment materials interfere with bonds between the first and second components 340a, 340b of the accommodating structure 340. In some embodiments, a hydrophobic coating is applied to the components 340a, 340b to be bonded when hydrophilic adhesives are used.

In some embodiments, second component 340b of the accommodating structure 340 includes a notch 389 or other cut out portion along all or a portion of the perimeter of the posterior side of the second optical component 350. In some embodiments, the notch 389 is filleted or otherwise curved.

Referring again to FIG. 3C, in some embodiments the accommodating structure 340 can include one or more sharp interior edges or corners. For example, the folded portion of the second component 340b between the outer bellows 303 and a second optical component 350 can include one or more sharp internal corners 393. In some embodiments, the sharp internal corners 393 have internal radii close to or equal to zero inches. In some embodiments, a posterior and of the outer bellows 303 includes one or more internal sharp corners 394. The one or more internal sharp corners 394 can have radii of curvature close to or equal to zero inches. The corners 393, 394 can provide point to mechanical weakness which may facilitate efficient collapse of the outer bellows 303 and/or other portions of the bellows of the AIOL 300 when radially inward forces applied to the outer bellows 303 from the capsule. Efficient collapse of the outer bellows 303 is expected to improve performance of the accommodating structure 340 by increasing fluid flow between the outer bellows and the inner fluid chamber 305.

In some embodiments, as illustrated in FIG. 3D, a height (e.g., as measured in the anterior-posterior direction) of the outer bellows 303' can be shortened, as compared to other embodiments of the present disclosure. For example, the height H5 of the outer bellows 303' can be less than 75%, less than 70%, less than 65%, and/or less than 60% of the height H6 of the accommodating structure 340'. In some embodiments, height H5 of the outer bellows 303' is approximately 70% of the height H6 of the accommodating structure 340'. As an example comparison, the height H7 of the outer bellows 303 of the accommodating structure 340 of FIG. 3C can be between approximately 75%-85% the height H8 of the accommodating structure 340. In some embodiments, shortening the height H5 of the outer bellows 303' is expected to improve the fit of the outer bellows 303' in the radially outward portions of the capsule. Precise ratios of the height H5 of the outer bellows 303' to the height H6 of the accommodating structure 340 can be adjusted to conform to the capsule of a given patient and to improve the fit of the AIOL 300 within the capsule.

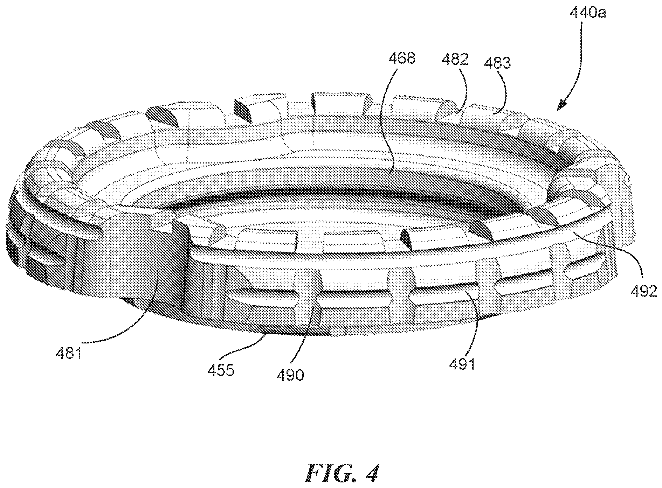

FIG. 4 illustrates a first portion 440a of accommodating structure of an AIOL having many or all of the same features of AIOLs 100, 200, 300 described above with respect to FIGS. 1A-3D. For example, like reference numbers between FIGS. 3A-3C and FIGS. 1A-2E indicate identical or similar features (e.g., indentation 282 v. indentation 382 v. indentation 482). In some embodiments, the first portion 440a (e.g., the anterior lens component) includes one or more channels 490 in a radially outward surface that extends in the anterior-posterior direction. In some embodiments, the first portion 440a includes one or more lateral channels 491 extending at an oblique angle with respect to the channels 490. For example, lateral channels 491 can extend perpendicularly with respect to the channels 490 and intersect the channels 490. In some embodiments, the lateral channels 491 extend around all or some of the outer perimeter of the first portion 440a in a direction tangential to the radially outward surface of the first portion 440a and perpendicular to the central optical axis of the first portion 440a. The first portion 440a can also include one or more secondary lateral channels 492 in the outer surface of the first portion 440a. For example, one or more secondary lateral channels 492 can extend parallel to and/or anterior of the first lateral channels 491 and can be spaced from the first lateral channels 491 in a direction parallel to the central optical axis of the first portion 440a. In some embodiments, the one or more secondary lateral channels 492 extend posterior to the lateral channels 491. In some embodiments, secondary lateral channels 492 extend in a direction oblique to the direction of the first lateral channels 491 and/or to the direction of the channels 490. The use of channels 490, first lateral channels 491 and/or second lateral channels 492 can improve fluid flow and/or aqueous circulation within an eye capsule past and/or around the AIOL. Improved fluid flow can improve the hydration, inhibit capsule fibrosis and maintain performance of the native eye capsule during accommodation.

In some embodiments, the bellows and/or other portions of an AIOL can include dimples, rivulets, channels, and/or other surface deformations configured to facilitate passage of fluid between the AIOL in the native eye capsule of the patient. The surface deformations can include temples, indentations, scoring, and/or other features. Preferably, the surface deformations have a depth on the order of the thickness of the eye capsule to reduce or eliminate the risk of the eye capsule filling or substantially filling the surface deformations. In some embodiments, the surface deformations of the AIOLs described herein can be arranged and configured to direct fluid toward the notches (e.g., notches 181, 281, 381, 481) to increase fluid flow past the AIOLs. Use of such surface deformations is expected to encourage and/or facilitate distribution of fluid over all or most of the interior surface of the eye capsule.

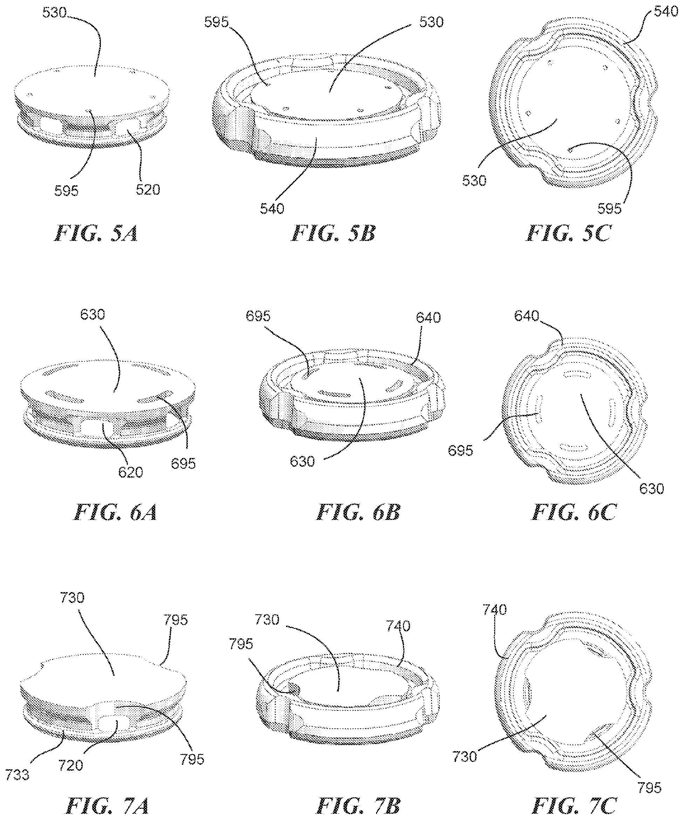

FIGS. 5A-7C illustrate affixed lenses 530, 630, 730 having flushing features configured to facilitate passage of OVD or other fluid(s) out from the space between the fixed lenses and the accommodating lenses of the AIOLs. For example, as illustrated in FIGS. 5A-5C, the fixed lens 530 can include one or more apertures 595 extending through the lens component. The apertures 595 can facilitate passage of one or both of OVD and natural eye fluid through the fixed lens 530. In some embodiments, as illustrated in FIGS. 6A-6C, the fixed lens 630 can include one or more slots 695 through the lens component. The slots 695 can facilitate passage of one or both of OVD and natural eye fluid through the fixed lens 630. In some embodiments, the slots 695 have an arcuate length between 20°-60°, between 25°-70°, between 30°-55°, and/or between 40°-50° is measured with respect to the central optical axis of the fixed lens 630. In some embodiments, the slots 695 have an arcuate length of approximately 45°.

In as the embodiment illustrated in FIGS. 7A-7C, a fixed lens 730 can include one or more cutouts 795 from an outer perimeter of the fixed lens 730. The cutouts 795 can be, for example, notches cut into or otherwise formed in an outer perimeter of the fixed lens 730. In some embodiments, the fixed lens 730 includes one cutout 795. The fixed lens 730 can include two or more cutouts 795 (e.g., two, three, four, or more cutouts 795). One or more of the cutouts 795 can extend into the fixed lens 730 in a direction perpendicular to the central optical axis of the fixed lens 730 to a depth of approximately 20% of the radius of the fixed lens 730. In some embodiments, the above-described depth of the cutout(s) 795 is between 5%-40%, between 10%-30%, and/or between 20%-50% of the radius of the fixed lens 730. The cutouts 795 may have an arcuate length between 20°-60°, between 25°-70°, between 30°-55°, and/or between 40°-50° is measured with respect to the central optical axis of the fixed lens 730. The cutouts 795 can extend through the entire fixed lens 730 and the anterior-posterior direction. In some embodiments, the cutouts 795 do not extend through a posterior flange 733 of the skirt of the fixed lens 730. Maintaining an annular posterior flange 733 on the skirt of the fixed lens is expected to improve the structural integrity of the accommodating structure 740. In some embodiments, the fixed lens can include one of the above described flushing features 595, 695, 795 or a combination of two or more of the flushing features 595, 695, 795.

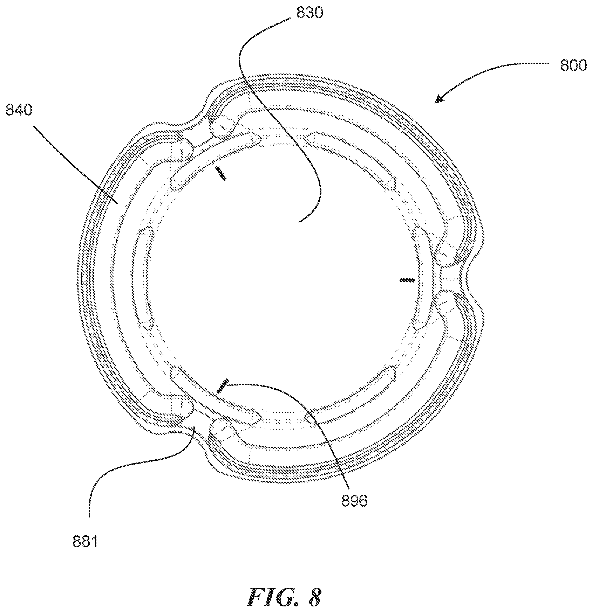

FIG. 8 illustrates an AIOL 800 configured in accordance with another embodiment of the present technology. The AIOL 800 has many or all of the same features of the AIOL 100 described above. In some embodiments, for example, the accommodating structure 840 of the AIOL 800 is identical to or similar to the accommodating structure 140 of AIOL 100. The accommodating structure 840 of the AIOL 800 can have markings 896 positioned on one or both of the first and second optical components of the accommodating structure 840. The markings 896 can be viewable from outside of the patient's eye and or through the fixed lens 830 of the AIOL 800. The markings 896 allow a surgeon or other medical personnel to locate the flow-through features 881 (e.g., indentations) or other specific features of the AIOL 800. For example, the AIOL 800 can include a marking 896 aligned with each of the flow-through features 881. Preferably, the markings 896 are positioned at or near a perimeter of the optical portion of the AIOL to avoid optical distortions for the patient. Accurately and reliably locating the flow-through features 881 can allow medical personnel to position cannulas or other flushing devices in orientations to efficiently flush OVD or other materials from the eye capsule posterior to the AIOL 800. For example, knowing the position of the flow-through features 881 can allow medical personnel to direct flushing fluid toward the flow-through features 881 when flushing the portion of the lens capsule posterior of the AIOL 800.

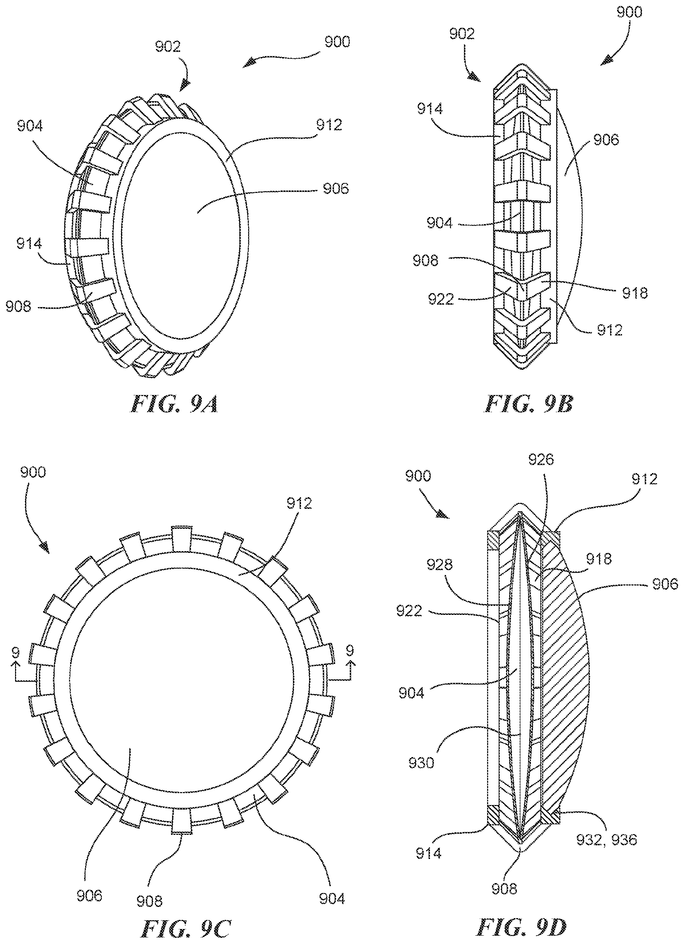

FIGS. 9A-9D illustrate an embodiment of an AIOL 900 configured in accordance with another embodiment of the present technology. The AIOL 900 includes a haptic structure 902 and an accommodating lens 904 positioned at least partially within the haptic structure 902. In some embodiments, the AIOL 900 includes a fixed lens 906. The fixed lens 906 can be connected to an anterior side of the haptic structure 902. In some embodiments, the AIOL 900 is oriented such that the fixed lens 906 is connected to the posterior side of the haptic structure 902. The haptic structure 902, as explained in more detail below, can be configured to translate forces from the lens capsule of an eye to the accommodating lens 904 and/or to the fixed lens 906.

In some embodiments, as illustrated in FIG. 9B, the haptic structure 902 can include individual spring elements 908. The individual spring elements 908 can be distributed in a generally circular elliptical or other-shaped array about a perimeter of the haptic structure 902 (e.g., about a center or optical axis of the haptic structure 902). The spring elements 908 can be configured to permit passage of fluid around and past the haptic structure 902 (e.g., between the spring elements 908) when the haptic structure 902 is implanted in the eye of the patient.

In some embodiments, the spring elements 908 have a first end connected to a first frame or ring 912 (e.g., an anterior ring) and a second end connected to a second frame or ring 914 (e.g., a posterior ring). The first frame 912 can be positioned at an anterior end of the haptic structure 902 and the second frame 914 can be positioned at a posterior end of the haptic structure 902. In some embodiments, one or both of the first and second frames 912, 914 have a ring-shape, are annular, are toroidal, and/or define a central opening.

In some embodiments, spring elements 908 have a curved shape. Alternatively, the spring elements 908 may have a bent and/or chevron shape. For example, the spring elements 908 can include anterior portions 918 connected to the first frame 912 and posterior portions 922 connected to the second frame 914. The anterior and posterior portions 918, 922 can be connected to each other at a corner of each of the spring elements 908. In some embodiments, the corners of the spring elements 908 are coplanar with each other on a plane perpendicular to an optical axis of the AIOL 900. The corners of the spring elements 908 can define a radially-outer perimeter of the haptic structure 902 with respect to the optical axis of the AIOL 900. The corners can be, for example, hinges about which the anterior and posterior portions 918, 922 can rotate with respect to each other (e.g., in response to forces upon the spring elements 908 from the native eye capsule in which the AIOL 900 is implanted).

As illustrated in FIG. 9D, the fixed lens 906 can be coupled with the haptic structure 902. The fixed lens 906, for example, can be connected to the first frame 912. In other embodiments, however, the fixed lens 906 is connected to the second frame 914. The fixed lens 906 is preferably removably couplable to one of the frames 912, 914. In some embodiments, fixed lens 906 can be coupled to and/or removed from one of the frames 912, 914 after the haptic structure 902 is implanted in the ocular lens of a patient. In some embodiments, the first frame 912 (and/or the second frame 914) includes a recess, channel, detent, or other structure configured to receive the fixed lens 906. For example, first frame 912 can include a channel 932 (e.g., an annular channel) in a radially inward surface of the first frame 912. In some embodiments, first frame 912 includes two or more discrete and separate channels in the radially inward surface of the first frame 912. The outer perimeter 936 of the fixed lens 906 can be configured to releasably couple with the one or more channels (e.g., the channel 932) in the radially inward surface of the first frame 912. In some embodiments, the outer perimeter 936 of the fixed lens includes one or more channels configured to receive ridges, protrusions, and/or other features on an inner perimeter of the first frame 912 for the second frame 914.

The accommodating lens 904 can include an anterior lens portion 926 and a posterior lens portion 928. The accommodating lens 904 (e.g., a fluid chamber) can be filled with a fluid between the anterior lens portion 926 and the posterior lens portion 928. The fluid may comprise a solution, an oil, a silicone oil, a solution of dextran, a solution of high molecular weight dextran, and/or a solution of another high molecular weight compound.

In some embodiments, the anterior and posterior lens portions 926, 928 are joined to each other at the perimeters 930 of each lens portion. In such embodiments, the accommodating lens 904 can be removably coupled with the haptic structure 902. For example, the accommodating lens 904 can be inserted into the haptic structure 902 through the opening in the anterior frame 912 or through the opening in the posterior frame 914. The accommodating lens 904 can be held in place with a haptic structure 902 via an interface between the accommodating lens 904 and the radially inward sides of the spring elements 908. For example, the perimeter of the accommodating lens 904 can interface with the inside corners of the spring elements 908.

Spring elements 908 can be configured to impart a radially inward force on the accommodating lens 904 when radially inward force is imparted on the spring elements 908. Radial compression of the accommodating lens 904 by the spring elements 908 can force the anterior and posterior lens portions 926, 928 away from each other. Relaxation of radial compression of the accommodating lens 904 by the spring elements 908 can allow the anterior and posterior lens portions 926, 928 to return toward each other. Movement of the anterior and posterior lens portions 926, 928 toward and away from each other in a direction parallel to the optical axis of the AIOL 900 can allow for changes in the optical power of the accommodating lens 904. The first and second frames 912, 914 can be configured to move apart from each other (e.g., a distance between the first and second frames 912, 914 can increase) in response to radial compressive force on the spring elements 908.

In some embodiments, the anterior lens portion 926 is integral with, formed with, or otherwise connected to the anterior portions 918 of the spring elements 908. The posterior lens portion 928 of the accommodating lens 904 can be integral with, formed with, or otherwise connected to the posterior portions 922 of the spring elements 908. In such embodiments, the anterior and posterior lens portions 926, 928 can be joined to each other as the anterior and posterior portions 918, 922 are joined to each other. Joining these respective portions to each other can include bonding, adhering, welding, friction fitting, and/or some other method of joining.

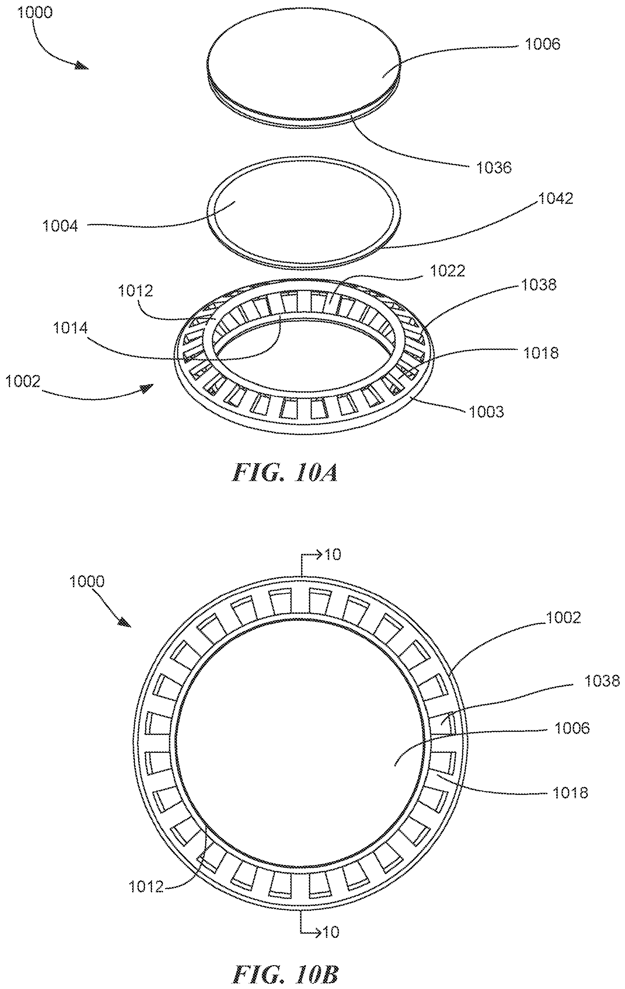

FIGS. 10A-10H illustrate an AIOL 1000 configured in accordance with another embodiment of the present technology. The AIOL 1000 can include a number of features similar to or the same as the features of the AIOL 900 described above. For example, like numbered-components can be similar to or the same as each other (e.g., fixed lens 906 v. fixed lens 1006). The AIOL 1000 includes a haptic structure 1002 configured to be removably coupled to one or both of the fixed lens 1006 and an accommodating lens 1004. The accommodating lens 1004 can include anterior and posterior lens portions similar to or the same as the anterior and posterior lens portions 926, 928 described above with respect to FIG. 9D, as well as a fluid chamber between the anterior and posterior lens portions. The haptic structure 1002 can be configured to contact the lens capsule of the patient when implanted in the lens capsule. The haptic structure 1002 of AIOL 1000 is configured to translate mechanical forces from the lens capsule to one or both of the accommodating lens 1004 and the fixed lens 1006.

The haptic structure 1002, as illustrated in FIG. 10A, can include anterior portions 1018 and posterior portions 1022 connected to each other at or near an outer perimeter of the haptic structure 1002. In some embodiments, the anterior portions 1018 of the haptic structure 1002 are similar to or the same in size as the posterior portions 1022. In some embodiments, the anterior portions 1018 and posterior portions 1022 of the haptic structure 1002 are connected to an outer ring 1003 of the haptic structure 1002. The outer ring 1003 can be a continuous annular ring around the entire perimeter of the haptic structure 1002 or broken ring separated into segments along the perimeter of the haptic structure 1002. The spaces between the anterior portions 1018 around the circumference of the haptic structure 1002 and between the posterior portions 1022 around the circumference of the haptic structure 1002 can define windows 1038 through the haptic structure 1002. These windows 1038 can permit passage of fluid through the haptic structure 1002 when the haptic structure 1002 is implanted in the eye of a patient.

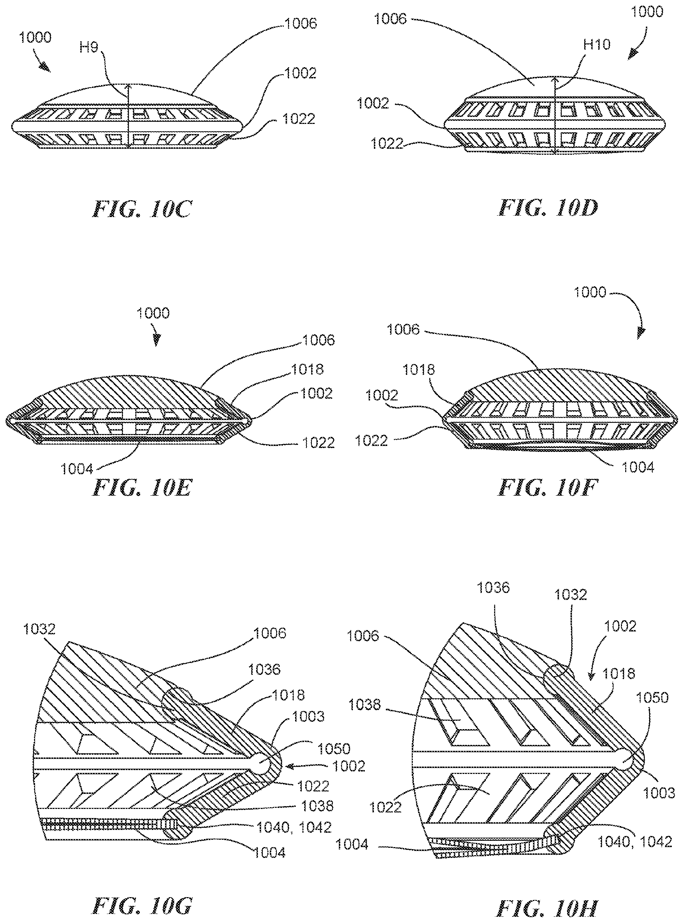

As best seen in FIG. 10G, the outer perimeter of the fixed lens 1006 includes a recess 1036 configured to releasably receive the anterior ring 1012 of the anterior portion 1018 and the haptic structure 1002. The recess 1036 can have a curved, elliptical, and/or semicircular shape as observed in a cut-plane parallel to and passing through the optical axis of the AIOL 1000. The interface between the anterior portion 1018 of the haptic structure 1002 and the perimeter of the fixed lens 1006 can permit relative tilting the anterior portion 1018 with respect to the fixed lens 1006 and the cut-plane illustrated in FIGS. 10E-10H. For example, the respective rounded surfaces of the recess 1036 and anterior ring 1012 of the anterior portion 1018 can allow for such tilting. Tilting of the anterior portion 1018 can, in turn, cause tilting of the perimeter of the accommodating lens 1004.

In some embodiments, the posterior portion 1022 of the haptic structure 1002 includes a recess 1040 (e.g., in the posterior ring 1014) configured to receive a portion (e.g., a perimeter 1042) of the accommodating lens 1004. Perimeter 1042 of the accommodating lens 1004 can be flexible or semi-flexible. In some embodiments, the interface between the posterior portion 1022 of the haptic structure 1002 and the accommodating lens 1004 can form a hinge about which the accommodating lens 1004 and/or posterior portion 1022 of the haptic structure 1002 rotate (e.g., tilt) with respect to each other in the cut-plane illustrated in FIGS. 10E-10H (e.g., a cut-plane passing through and parallel to the central optical axis of the AIOL 1000). As the accommodating lens 1004 rotates (e.g., in the posterior direction and/or away from the outer ring/hinge 1003), the radially-outward portion of the lens 1004 (e.g., at or near the periphery of the lens 1004) can compress in the axial direction, forcing fluid radially inward within the accommodating lens 1004. This radially-inward flow of fluid can expand the radially inward portions of the accommodating lens 1004, thereby changing the optic power of the accommodating lens 1004.

In some embodiments, the haptic structure 1002 can include a weakened portion 1050 configured to facilitate flexing and/or rotation of the posterior portion 1022 of the haptic structure 1002 with respect to the anterior portion 1018, and vice-versa. The weakened portion 1050 can be, for example one or more cuts, recesses, channels, or other features in a radially inward portion of the outer ring 1003, posterior portion 1022, and/or anterior portion 1018. In some embodiments, the radially outward portion (e.g., the periphery) of the haptic structure 1002 includes one or more indentations, cuts, recesses, channels, and/or other features configured to facilitate fluid flow around and past the radially-outward portion of the haptic structure 1002. While the various features of the AIOL 1000 are described above with the accommodating lens 1004 connected to the posterior ring 1014, the AIOL 1000 could be flipped such that the posterior 1014 ring is on the anterior side and the anterior ring 1018 is on the posterior side of the AIOL 1000.

FIGS. 10C, 10E, and 10G illustrate the AIOL 1000 in a non-accommodating configuration, and FIGS. 10D, 10F, and 10H illustrate the AIOL 1000 in an accommodating configuration. Put another way, FIGS. 10D, 10F, and 10H illustrate the AIOL 1000 in a configuration wherein radially-inward force is applied to the haptic structure 1002 (e.g., by the lens capsule of a patient). In the accommodated configuration, the haptic structure 1002 is radially compressed and axially (e.g., parallel to the optic axis of the AIOL 1000) extended. Axial extension of the haptic structure 1002 increases the axial height of the haptic structure 1002 from a non-accommodated height H9 (FIG. 10C) to an accommodated height H10 (FIG. 10D). In some embodiments, the accommodated height H10 of the haptic structure 1002 is between 10%-50%, between 20%-40%, between 30%-35% greater than the non-accommodated height H9 of the haptic structure 1002. During the transition between accommodating and non-accommodating configurations, the haptic structure 1002 and/or accommodating lens 1004 can hinge and/or rotate with respect to each other, as described above.

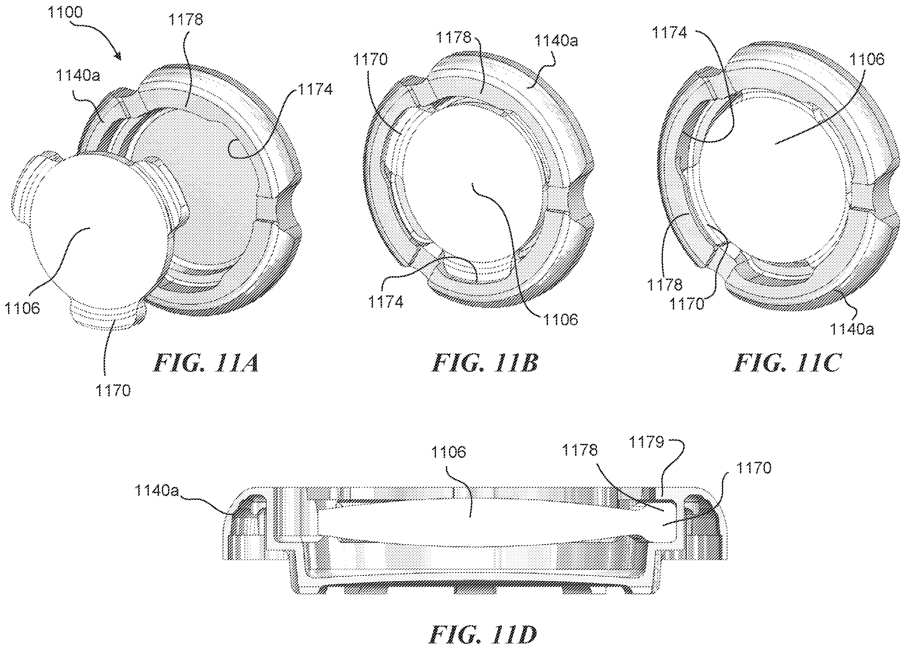

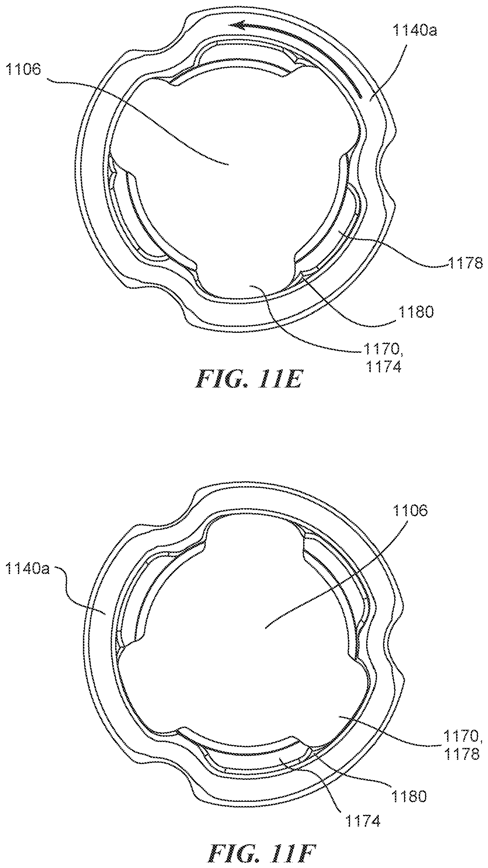

FIGS. 11A-11D illustrate an AIOL 1100 configured in accordance with another embodiment of the present technology. The AIOL 1100 can include a number of features similar to or the same as the features of the AIOL 1000 described above. Accordingly, like numbered-components can be similar to or the same as each other (e.g., fixed lens 1006 v. fixed lens 1106). The AIOL 1100 can include a fixed lens 1106 having one or more mating structures 1170 extending from an optical portion of the fixed lens 1106. The mating structures 1170 can be, for example, fins, protrusions, flanges, extensions, or other structures extending from the optical portion of the fixed lens 1106 in a direction perpendicular to or generally perpendicular to an optical axis of the fixed lens 1106. In the illustrated embodiments, the fixed lens 1106 includes three mating structures 1170 evenly distributed in a circumferential pattern around the perimeter of the optical portion of the fixed lens 1106. In some embodiments, more or fewer mating structures are used, and patterns other than even circumferential distribution may be used. The first component 1140a (e.g., an anterior component) of the accommodating structure (e.g., the base lens) of the AIOL 1100 can include one or more receiving structures 1174 configured to receive and releasably mate with the mating structures 1170 of the fixed lens 1106. While the second component of the accommodating structure is not illustrated, it can be identical or similar to one or more of the second components 140b, 240b, 340b described above. One or more of the receiving structures 1174 can be, for example, a slot or other indentation configured to receive corresponding mating structures 1170 (e.g., in a direction parallel to the optical axis of the AIOL 1100), as illustrated in FIG. 11B. The first component 1140a can include a retaining structure 1178 (e.g., one retaining structure 1178 for each receiving structure 1174) configured to secure the fixed lens 1106 to the first component 1140a when the fixed lens 1106 is fully coupled with the first component 1140a. The retaining structures 1178 can be, for example, pockets, depressions, indentations, cavities, or other structures configured to retain at least a portion of the mating structures 1170 of the fixed lens 1106. These structures for mating with the fixed lens may singular or plural structures. In the illustrated embodiment, the retaining structures 1178 are circumferentially-extending cavities extending from the receiving structures 1174. After the mating structures 1170 are received in/through the receiving structures 1174 (e.g., slots), the fixed lens 1106 can be rotated (e.g., in a counterclockwise direction) such that the mating structures 1170 are at least partially received and retained in the retaining structures 1178 (FIGS. 11C-11D).

The retaining structures 1178 can be configured to inhibit or prevent inadvertent decoupling of the fixed lens 1106 from the first component 1140a. For example, the retaining structures 1178 can include anterior walls 1179 (FIG. 11D) positioned to inhibit or otherwise interfere with movement of the mating structures 1170 when the mating structures 1170 are positioned at least partially within the retaining structures 1178.

In some embodiments, the mating structures 1170 are sized/shaped to deflect a portion of the retaining structures 1178 and/or increase friction between the mating structures and the retaining structures 1178 when the mating structures are positioned at least partially within the retaining structures 1178. For example, a portion of the mating structures 1170 can be larger in one or more dimensions (e.g., parallel to and/or perpendicular to the optical axis of the AIOL 1100) than the retaining structures 1178 such that one or more of the retaining structures 1178 are deflected and/or deformed when a mating structure 1170 is received therein.

FIGS. 11E-11H illustrate additional features that may be used in combination with the above-described AIOL 1100. For example, referring to FIGS. 11E-11F, the first component 1140a can include a bump 1180, protrusion, or other structure configured to interfere with rotation of the fixed lens 1106 when the mating structures 1170 are positioned at least partially within the retaining structures 1178. The bumps 1180 can be positioned on a radially-inward wall of the first component 1140a between the receiving structure 1174 and the retaining structure 1178. The bumps 1180 can be configured to deflect outward as the mating structures 1170 move from the receiving structures 1174 to the retaining structures 1178 (e.g., as the fixed lens 1106 is rotated with respect to the first component 1140a, as indicated by the arcuate arrow in FIG. 11E). Once the mating structures 1170 pass the bumps 1180, the bumps 1180 can deflect radially inward to interfere with rotation of the mating structures 1170 back to the receiving structures 1174.

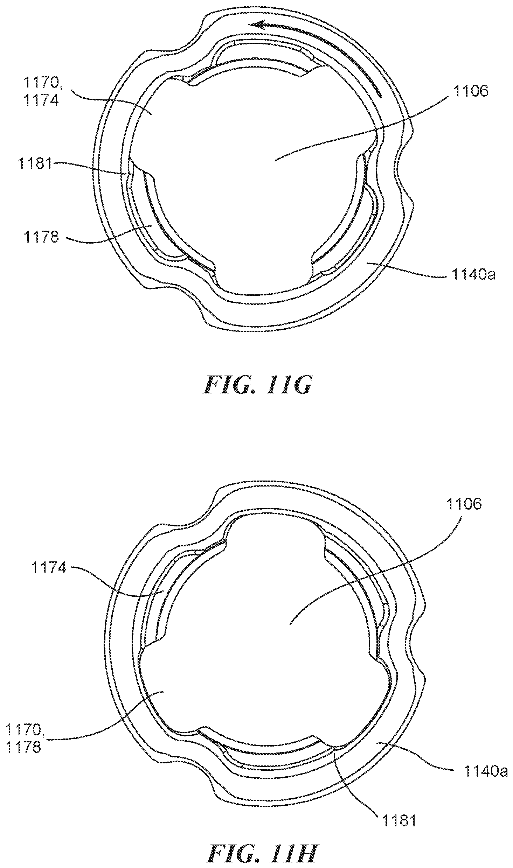

FIGS. 11G and 11H illustrate a feature that can be used instead of, or in addition to the above-described bump 1180. Specifically, the receiving structure 1174 can have a radius (e.g., as measured perpendicular to the optical axis of the AIOL 1100) that is smaller than a radius of the retaining structure 1178. The radius of the receiving structure 1174 can be smaller than the radius of the mating structures 1170 of the fixed lens 1106. In some embodiments, when the fixed lens 1106, or the mating structures 1170 thereof, are received in the receiving structures 1174, the mating structures 1170 can deflect the receiving structures 1174 in a radially-outward direction. When the mating structures 1170 are moved into the retaining structures 1178 (see, e.g., the arcuate arrow in FIG. 11G), the receiving structures 1174 can return to their undeflected positions. Steps 1181 between the radius of the receiving structures 1174 and the radius of the retaining structures 1178 are positioned to inhibit or prevent inadvertent movement of the mating structures 1170 from the retaining structures 1178 to the receiving structures 1174.

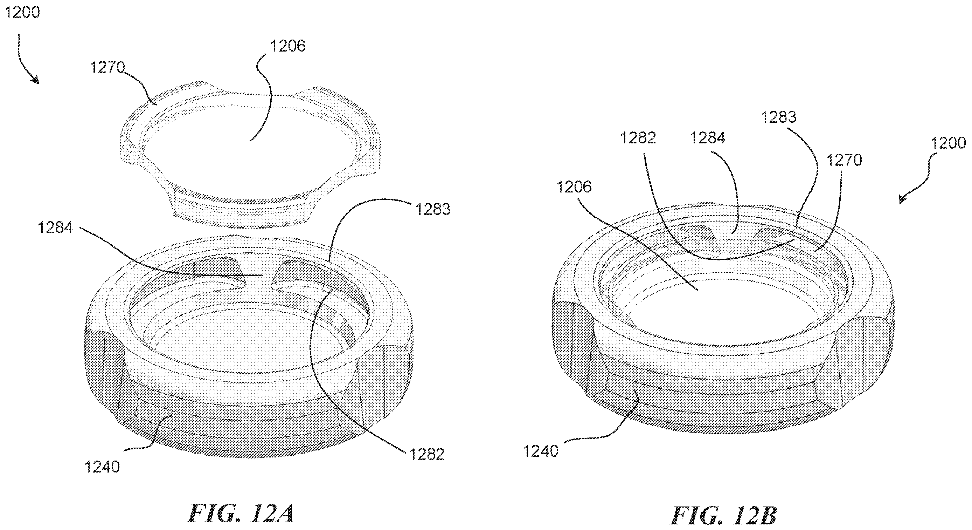

FIGS 12A-12B illustrate an AIOL 1200 configured in accordance with another embodiment of the present technology. The AIOL 1200 can include a number of features similar to or the same as the features of the above-described AIOLs (e.g., AIOLs 1000 and 1100). Thus, like numbered-components can be similar to or the same as each other (e.g., fixed lens 1106 v. fixed lens 1206). The accommodating structure 1240 of the AIOL 1200 can include a plurality of cavities 1282 or other cavities configured to releasably receive mating structures 1270 (e.g., fins, protrusions, flanges, or other mating structures) of the fixed lens 1206. The cavities 1282 can include anterior walls 1283. The anterior walls 1283 can be configured to bend, deflect, and/or otherwise deform to allow the mating structures 1270 of the fixed lens 1206 to pass at least partially into and out of the cavities 1282. The accommodating structure 1240 can include interior walls 1284 separating the cavities 1282. The interior walls 1284 can inhibit or prevent free rotation of the fixed lens 1206 when the fixed lens 1206 is mated with the accommodating structure 1240 (as shown in FIG. 12B).

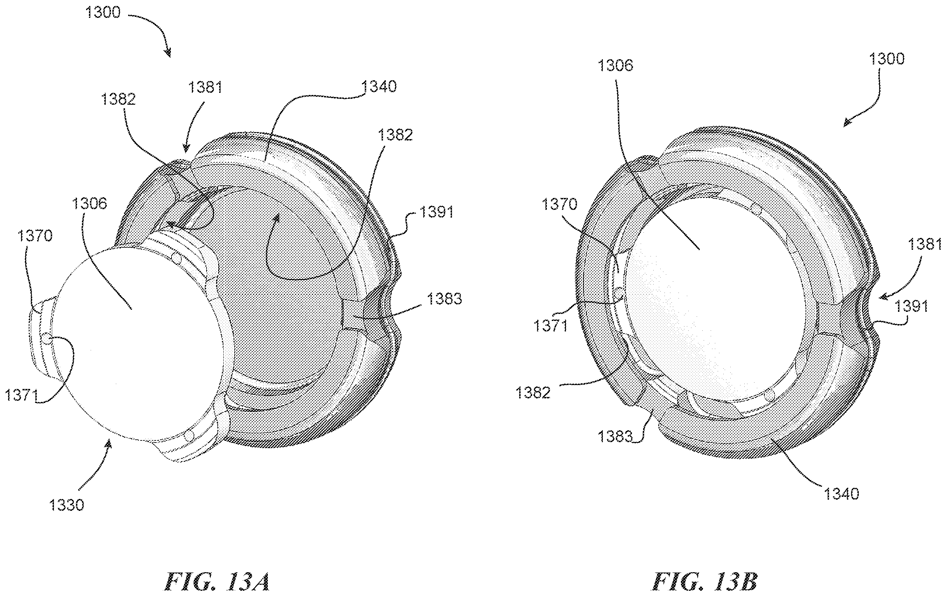

FIGS. 13A-13D illustrate an AIOL 1300 configured in accordance with another embodiment of the present technology. The AIOL 1300 can include a number features similar to or the same as the features of AIOLs 100, 200, 300, 1100, and 1200 described above. Accordingly, like numbered components can be similar to or the same as each other (e.g., cavities 1282 v. cavities 1382, fixed lens 1206 v. fixed lens 1306, etc.). Referring to FIGS. 13A and 13B, the fixed lens assembly 1330 can include a lens portion 1306 (e.g., a fixed lens) and one or more tabs 1370 extending radially outward from the lens portion 1306. One or more holes 1371 can extend at least partially through the one or more tabs 1370. The holes 1371 (e.g., or indentation) can be engaged by a surgical tool to manipulate the fixed lens assembly 1330 during implantation and/or removal of the fixed lens assembly 1330. In some embodiments, the holes 1371 are arranged in pairs. In some embodiments, the holes 1371 are distributed in a circumferential pattern. In some embodiments, one or more of the holes 1371 has a different size (e.g., width or diameter) and/or shape than other holes 1371. For example, each of the holes 1371 may have a different size than each of the other holes 1371. Using holes of varying size can allow for further visual confirmation of the rotational alignment of the fixed lens assembly 1330 (e.g., about the optical axis of the AIOL 1300). Confirming the alignment/orientation of the fixed lens assembly 1330 can reduce the risk that a toric lens or other non-annularly-symmetric fixed lens 1306 is improperly oriented with respect to the base lens 1340 and/or native eye capsule into which the AIOL 1300 is implanted. In some embodiments, the fixed lens assembly 1330 can include additional visual markers (e.g., similar to or the same as the markings 896 described above with respect to FIG. 8) to indicate an orientation of the fixed lens assembly 1330 with respect to the base lens 1340 and/or the native eye capsule.

The AIOL 1300 further comprises a base lens 1340 (e.g., accommodating structure). As best seen in FIG. 13A, the base lens 1340 can include one or more cavities 1382 or other mating structures configured to releasably couple with the one or more tabs 1370 of the fixed lens assembly 1330. The cavities 1382 can open toward an optical axis of the base lens 1340. In some embodiments, there are three cavities 1382 to receive three tabs 1370 of the fixed lens assembly 1330. The cavities 1382 can be positioned in portions of the base lens 1340 and separated from each other by the flow-through features 1381 and/or walls adjacent the indentations 1383 of the base lens 1340. The indentations 1383 can be aligned with flow-through features 1381 of the base lens 1340 having similar or identical features to the flow-through features 181 described above. The indentations 1383 and/or flow-through features 1381 can facilitate flow of fluid around the outer perimeter of the base lens 1340 when the base lens 1340 is implanted in the eye capsule of a patient. When the AIOL is assembled, the tabs 1370, fixed lens 1306, and/or the cavities 1382 can be coplanar with each other.

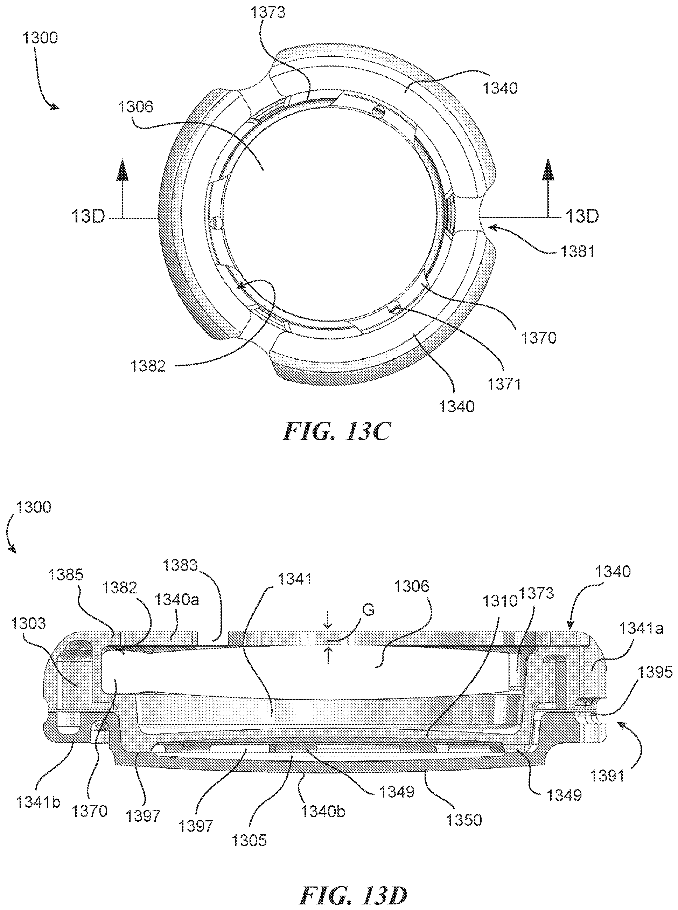

The base lens 1340 can include an outer channel 1391 on a radially-outer surface of the base lens 1340 (e.g., on the anterior base lens component 1340a and/or posterior base lens component 1340b, as illustrated in FIG. 13D). The outer channel 1391 can extend around the entire perimeter of the base lens 1340. In some embodiments, the outer channel 1391 extends along a seam 1395 (FIG. 13D) between the anterior and posterior base lens components 1340a, 1340b. The outer channel 1391 can facilitate increased fluid flow and/or flow of OVD around the outside of the base lens 1340 when the base lens is implanted in the eye capsule of a patient. In some embodiments, the outer channel 1391 can reduce the likelihood that the base lens 1340 adheres to the inner wall of the eye capsule when implanted.

FIG. 13C is a plan view of the anterior face of the AIOL 1300, and FIG. 13D illustrates a lateral cross-sectional view of the AIOL 1300 taken along the cut-plane 13D-13D of Fig. 13C. As best seen in FIG. 13D, the cavities 1382 can be at least partially defined by anterior flanges 1385. The flanges 1385 can overlap the tabs 1370 in a direction parallel to the optical axis of the of the base lens 1340 when the tabs 1370 are positioned within the cavities 1382. The flanges 1385 can inhibit or prevent inadvertent (e.g., under the forces of the native eye capsule) decoupling of the fixed lens assembly 1330 from the base lens 1340.

In some embodiments, the circumferential spaces between the tabs 1370 can at least partially define passages 1373 between the fixed lens 1306 and the base lens 1340 when the fixed lens assembly 1330 is coupled to the base lens 1340. The passages 1373 can permit fluid (e.g., aqueous humor) to pass into and out from a chamber 1341 between the fixed lens 1306 and the optical portion 1310 of the anterior base lens component 1340a.

As illustrated in FIG. 13D, the haptic reservoir 1303 of the base lens 1340 can be defined at least in part by the haptic portions 1341a, 1341b of the anterior and posterior base lens components 1340a, 1340b, respectively. The haptic reservoir 1303 can be in fluid communication with a fluid chamber 1305 between the optical portion 1310 of the anterior base lens component 1340a and the optical portion 1350 of the posterior base lens component 1340b. This fluid communication can be facilitated by one or more fluid flow paths 1349. These fluid flow paths 1349 can be defined by gaps between protrusions 1397 (e.g., standoffs) along the perimeter of the optical portion 1310 of the anterior base lens component 1340a. These protrusions 1397 can be similar to or the same as the standoffs 290 described above with respect to FIGS. 2G and 2H. In some embodiments, the posterior base lens component 1340b includes protrusions or standoffs to form the fluid flow paths 1249. These protrusions can extend from a perimeter of the optical portion 1350 of the posterior base lens component 1340b and can be used in addition to or instead of the protrusions 1397 described above.

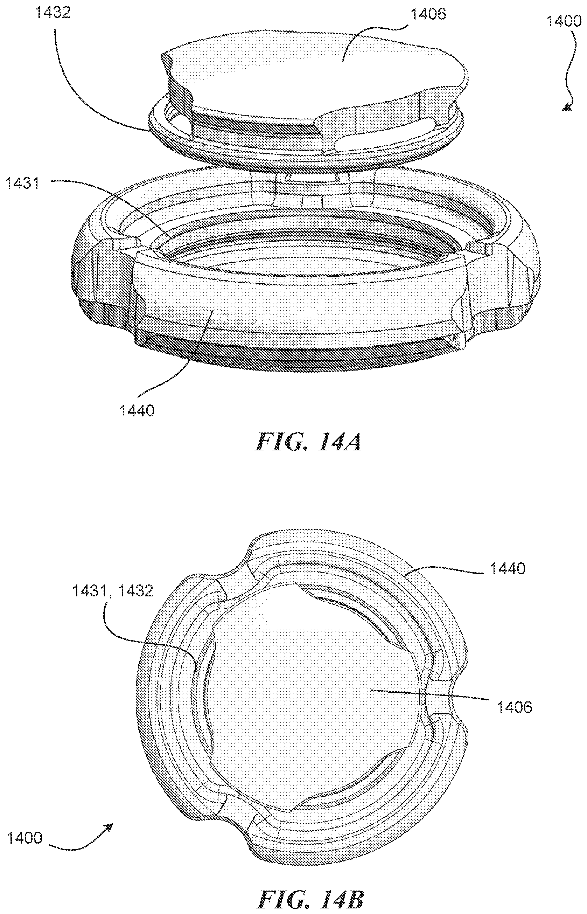

FIGS 14A-14B illustrate an AIOL 1400 configured in accordance with another embodiment of the present technology. The AIOL 1400 can include a number of features similar to or the same as the features of the AIOLs 100 and 200 described above. Accordingly, like numbered-components can be similar to or the same as each other (e.g., fixed lens 206 v. fixed lens 1406). The fixed lens 1406 of the AIOL 1400 can include a skirt 1432 sized and shaped to engage with an engagement feature 1431 of the accommodating structure 1440 (e.g., the base lens) in a manner similar to or the same as that described above with respect to the engagement feature 131 and skirt 132 of FIG. 1C-1D. In the embodiment shown in FIGS. 14A and 14B, all or a portion of the skirt 1432 (e.g., a visual marker) can be colored in a first color. All or a portion of the engagement feature 1431 (e.g., a visual marker) can be colored in a second color such that, when the fixed lens 1406 is mated with the accommodating structure 1440, a third color is observed from a position anterior of the AIOL 1400 (FIG. 14B) (e.g., through the engagement feature 1431). The third color is created from the overlap in the anterior-posterior direction of the skirt 1432 and the engagement feature 1431. For example, the first color can be blue and the second color can be yellow such that the third color is green. Other first and second color combinations (e.g., red-yellow, red-blue, etc.) can also be used. In some embodiments, the entire circumference of the skirt 1432 is colored with the first color so that full annular engagement between the fixed lens 1406 and the accommodating structure 1440 can be visually confirmed.

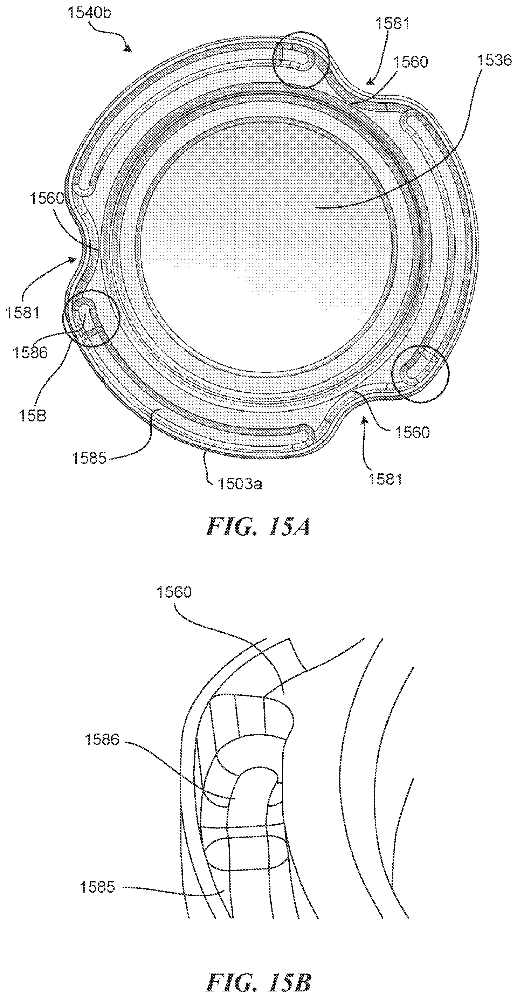

FIGS. 15A-15D illustrate a second component 1540b of an accommodating structure of an AIOL configured in accordance with another embodiment of the present technology. The second component 1540b can be used in combination with first components of the accommodating structures of the above-described AIOLs. Referring to FIGS. 15A and 15B, the second component 1540b can include thickened portions 1560 adjacent the flow-through features 1581 similar to the thickened portions 160 and flow-through features 181 described above in FIGS. 1A-1D with respect to AIOL 100. The second component 1540b can also include an outer bellow region 1503a between the thickened portions 1560 (e.g., in the circumferential direction) and at least partially surrounding an optical portion 1536 of the second component 1540b. The outer bellows regions 1503a can include posterior walls 1585 extending along all or a portion of the lengths of the outer bellows regions 1503a in the circumferential direction.

One or more of the posterior walls 1585 can include a filling portion 1586 or some other portion of thicker construction. The filling portion 1586 can be thicker (e.g., in a direction parallel to the optical axis of the optical portion 1536) than the remaining posterior walls 1585, and/or thinner than the thickened portions 1560. The filling portion(s) 1586 can be positioned, for example, at one or more of the ends (e.g., circumferential ends) of the posterior walls 1585 and/or adjacent the thickened portions 1560.

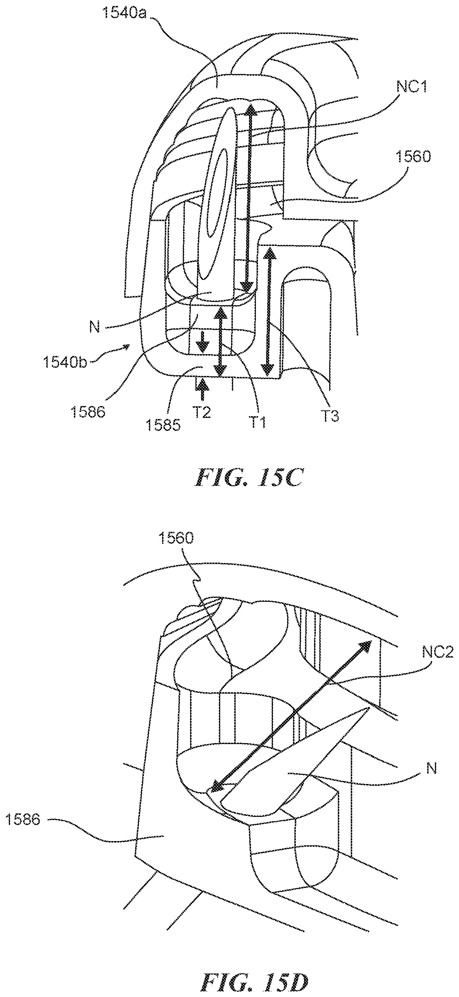

FIGS. 15C and 15D illustrate close-up cross-sectional views of an individual filling portion 1586 of the second component 1540b of FIG. 15A with a needle N extending therethrough. Referring to FIGS. 15C and 15D together, the filling portion 1586 can be configured to permit passage of needles N therethrough to fill the accommodating structure with optical fluid (not shown). The thickness T1 of the filling portion 1586 can be sufficient such that, when the needle N is removed from the accommodating structure, the material of the filling portion 1586 retains a seal around the needle N and inhibits or prevents escape of fluid when the needle N exits the accommodating structure. In some embodiments, for example, the thickness T1 of the filling portion 1586 is between 150%-200%, between 200%-300%, between 125%-400%, between 250%-500%, and/or between 110%-2500% of the thickness T2 of the posterior wall 1585. In some embodiments, the thickness T1 of the filling portion 1586 is between 25%-75%, between 10%-80%, between 40%-60%, between 50%-90%, and/or between 40%-70% of the thickness T3 of the thickened portions 1560. In some embodiments, the filling portion 1586 is approximately 2 millimeters (mm) thick, between 1.5-2.1 mm thick, between 1.9-2 mm thick, and/or between 1.75-2.2 mm thick.

Setting the thickness of the filling portion 1586 as described above can allow for a needle clearance NC1 (e.g., an amount of space between an anterior surface of the filling portion 1586 and the anterior wall of the first component 1540a of the AIOL, as illustrated in FIG. 15C) that is greater than the needle clearance provided by the thickened portion 1560. Increasing the needle clearance can allow for use of needle with steeper beveled ends than may otherwise be usable with a shorter clearance. In some embodiments, the needle clearance NC1 anterior of the filling portions 1586 is approximately 1.25 mm, between 1.1-1.5 mm, between 1.2-1.3 mm, and/or between 1-1.6 mm. The angled needle clearance NC2 (FIG. 15D) provided by the filling portions 1586 can be approximately 1.33 mm, between 1.1-1.4 mm, between 1.15-1.45 mm, and/or between 1.3-1.35 mm. In some embodiments, inclusion of the filling portions 1586 can reduce the risk of aberrations (e.g., non-circular flexure or other deformation) in the optical portion 1536 during accommodation.

The multipart AIOL devices described herein may be implanted by preparing the eye and removing the native lens from the capsule in any appropriate manner. The fluid-filled structure may then be placed in the capsule of the eye. The patient may then be evaluated for a base optical power and/or astigmatic correction, and a fixed lens is selected to provide the desired based power or astigmatic correction for the fluid-filled structure in the implanted state in the capsule of the eye. The specific fixed lens to provide the post-implant base power or astigmatic correction is then inserted into the previously implanted fluid-filled structure of the AIOL. The chosen fixed lens may then be coupled to the fluid-filled structure within the eye capsule. This is possible in the AIOLs of the present technology because the fixed lenses are attached to the anterior first component of the AIOLs. As described above, one or more of the fluid-filled accommodating structure or fixed lens may each be flexible such that they may be reconfigured (e.g., folded) to a reduced-profile delivery configuration for delivery into the lens capsule. In some instances, it may be required to make a further correction to the fixed portion after the time of the surgery. Such instance may occur anywhere from days to years after the surgery. At such times, the patient may return to the physician and the fixed lens may be replaced with a new fixed lens having a different optical power or other prescription. In such instances, the new prescription may be characterized prior to or after removal of the original fixed lens. In some instances, the new fixed lens may be fabricated and implanted at the time of the examination, in others the patient may return for implantation of the fixed lens sometime after the examination.

Several embodiments of the present technology are directed to a kit having an accommodating structure and a first fixed lens that has no optical base power. The kit can further include one or more second fixed lenses having various based powers or other optical properties. In practice, the accommodating structure can be implanted into the native eye capsule, and then the first fixed lens can be coupled to the accommodating structure. The optical properties of the implanted accommodating structure can then be assessed in situ with the first fixed lens in place to determine the desired optical properties of the fixed lens. If the optical properties of the assembled accommodating structure and first fixed lens without a base power are appropriate, then the system can remain implanted without additional changes. However, if a different base power or some other optical property is desired (e.g., toric or other asymmetrical optics), then the first fixed lens without a base power can be replaced with a second fixed lens having the desired optical properties based on the optical properties of the implanted accommodating portion with a fixed lens attached.

In some embodiments, the fixed portion of the AIOL may be fabricated from materials different from the accommodating portion. Such materials include hydrophilic or hydrophobic methacrylate or silicones and any other materials traditionally used in non-accommodating IOLs. The fixed lens may be fabricated from materials harder than those used for the accommodating portion. One or both of the accommodating portion/lens and the fixed portion/lens may be machined, cast molded (e.g., reactive cast molded), injected molded, and/or formed by other processes or combinations of processes. Any or all of the structures described herein may be constructed from a transparent or translucent material. For example, the above-described accommodating structures and fixed lenses can be constructed from transparent materials, even if they are illustrated as opaque in the associated figures.

Any of the features of the intraocular lens systems described herein may be combined with any of the features of the other intraocular lenses described herein and vice versa. Additionally, several specific examples of embodiments in accordance with the present technology are set forth below in the following examples.

### Conclusion

The above detailed description of embodiments of the technology are not intended to be exhaustive or to limit the technology to the precise form disclosed above. Although specific embodiments of, and examples for, the technology are described above for illustrative purposes, various equivalent modifications are possible within the scope of the technology as set forth in the claims. For example, any of the features of the AIOLs described herein may be combined with any of the features of the other AIOLs described herein and vice versa. Moreover, although steps are presented in a given order, alternative embodiments may perform steps in a different order. The various embodiments described herein may also be combined to provide further embodiments.

From the foregoing, it will be appreciated that specific embodiments of the technology have been described herein for purposes of illustration, but well-known structures and functions associated with AIOLs have not been shown or described in detail to avoid unnecessarily obscuring the description of the embodiments of the technology. Where the context permits, singular or plural terms may also include the plural or singular term, respectively.

Moreover, unless the word "or" is expressly limited to mean only a single item exclusive from the other items in reference to a list of two or more items, then the use of "or" in such a list is to be interpreted as including (a) any single item in the list, (b) all of the items in the list, or (c) any combination of the items in the list. Additionally, the term "comprising" is used throughout to mean including at least the recited feature(s) such that any greater number of the same feature and/or additional types of other features are not precluded. It will also be appreciated that specific embodiments have been described herein for purposes of illustration, but that various modifications may be made without deviating from the scope of the appended claims.

## Claims

1. An accommodating intraocular lens (AIOL) (1300) comprising:
a base lens (1340) having-
an anterior base lens component (1340a) having a first optical portion (1310) and a first haptic portion (1341a) at least partially surrounding the first optical portion;
an optical axis;
a posterior base lens component (1340b) coupled to the anterior base lens component (1340a) and having a second optical portion (1350) aligned with the first optical portion along the optical axis and a second haptic portion (1341b) at least partially surrounding the second optical portion and aligned with the first haptic portion in a direction parallel to the optical axis;
a plurality of mating structures (1382) formed in one or both of the anterior base lens component (1340a) and the posterior base lens component (1340b), the plurality of mating structures (1382) open toward the optical axis;
a plurality of flow-through features (1381) formed by the anterior base lens component (1340a) and the posterior base lens component (1340b), wherein each of the flow-through features (1381) separate individual ones of the mating structures (1382);
an optical chamber (1305) between the first optical portion (1310) and the second optical portion (1350); and
a haptic reservoir (1303) between the first haptic portion (1341a) and the second haptic portion (1341b), the haptic reservoir (1303) in fluid communication with the optical chamber (1305); and
a fixed lens configured to be removably coupled with the base lens, the fixed lens having-
a lens portion (1306) having an anterior face and a posterior face, the lens portion providing a fixed optical power, and
a plurality of tabs (1370) extending radially outward from the lens portion (1306),
wherein, when the fixed lens is removably coupled with the base lens (1340)-the lens portion (1306) is aligned with the first and second optical portions (1310, 1350), and
each tab (1370) is positioned within one of the plurality of mating structures (1382).

2. The AIOL (1300) of claim 1 wherein the anterior face of the fixed lens is positioned posterior of an anterior-most edge of the anterior base lens component (1340a) when the fixed lens is coupled to the base lens (1340).

3. The AIOL (1300) of claim 1, further comprising a hole (1371) through one of the plurality of tabs (1370), the hole (1371) being configured to receive a portion of a tool for manipulating the fixed lens with respect to the base lens (1340).

4. The AIOL (1300) of claim 1 wherein:
the anterior base lens component (1340a) includes a first annular mating portion surrounding the first optical portion (1310);
the posterior base lens component (1340b) includes a second annular mating portion surrounding the second optical portion (1350); and
the first annular mating portion includes a plurality of protrusions (1397) configured to mate with the second annular mating portion;
wherein circumferential gaps between the plurality of protrusions (1397) at least partially define fluid flow paths (1349) between the haptic reservoir (1303) and the optical chamber (1305).

5. The AIOL (1300) of claim 1 wherein:
the anterior base lens component (1340a) includes a first annular mating portion surrounding the first optical portion (1310);
the posterior base lens component (1340b) includes a second annular mating portion surrounding the second optical portion (1350); and
the second annular mating portion includes a plurality of protrusions (1397) configured to mate with the first annular mating portion;
wherein circumferential gaps between the plurality of protrusions (1397) at least partially define fluid flow paths (1349) between the haptic reservoir (1303) and the optical chamber (1305).

6. The AIOL (1300) of claim 1, further comprising one or more indentations (1383) in an anterior-most surface of the anterior base lens component (1340a).

7. The AIOL (1300) of claim 6, wherein each of the flow-through features (1381) is circumferentially aligned with one of the one or more indentations (1383).

8. The AIOL (1300) of claim 1, further comprising a fluid chamber (1341) between the fixed lens and the first optical portion (1310) of the anterior base lens component (1340a) when the fixed lens is coupled with the base lens (1340), wherein circumferential gaps between the tabs (1370) of the fixed lens allow fluid to pass around fixed lens into and out from the fluid chamber (1341).

9. The AIOL (1300) of claim 1, further comprising a channel (1391) in an outer wall of the base lens, the channel (1391) extending along an entire perimeter of the base lens (1340).

10. The AIOL (1300) of claim 9 wherein the channel (1391) is positioned at a seam (1395) between the anterior base lens component (1340a) and the posterior base lens component (1340b).

11. The AIOL (1300) of claim 1 wherein the plurality of mating structures (1382) comprise circumferentially-extending cavities configured to receive the plurality of tabs (1370).

12. The AIOL (1300) of claim 1, further comprising a plurality of receiving structures (1178) configured to receive and releasably mate with the plurality of tabs (1370) of the fixed lens.

13. The AIOL (1300) of claim 12 wherein each of the plurality of mating structures (1382) comprises a cavity extending circumferentially from one of the plurality of receiving structures (1178).

14. The AIOL (1300) of claim 12 wherein-
when the fixed lens is coupled to the base lens (1340), the plurality of tabs (1370) are received within the plurality of receiving structures (1178); and
after rotation of the fixed lens relative to the base lens (1370), the plurality of tabs (1370) are received within the plurality of mating structures (1382).

## Patentansprüche

1. Akkommodierende Intraokularlinse (AIOL) (1300), die Folgendes umfasst:
eine Basislinse (1340) mit-
einer vorderen Basislinsenkomponente (1340a) mit einem ersten optischen Teil (1310) und einem ersten haptischen Teil (1341a), der den ersten optischen Teil zumindest teilweise umgibt;
einer optischen Achse;
einer hinteren Basislinsenkomponente (1340b), die mit der vorderen Basislinsenkomponente (1340a) gekoppelt ist und einen entlang der optischen Achse mit dem ersten optischen Teil ausgerichteten zweiten optischen Teil (1350) sowie einen zweiten haptischen Teil (1341b) aufweist, der den zweiten optischen Teil zumindest teilweise umgibt und in einer Richtung parallel zur optischen Achse mit dem ersten haptischen Teil ausgerichtet ist;
mehrere Passstrukturen (1382), die in der vorderen Basislinsenkomponente (1340a) und/oder der hinteren Basislinsenkomponente (1340b) ausgebildet sind, wobei die mehreren Passstrukturen (1382) zur optischen Achse hin offen sind;
mehrere Durchflussmerkmale (1381), die von der vorderen Basislinsenkomponente (1340a) und der hinteren Basislinsenkomponente (1340b) gebildet werden, wobei die einzelnen Durchflussmerkmale (1381) einzelne der Passstrukturen (1382) voneinander trennen;
eine optische Kammer (1305) zwischen dem ersten optischen Teil (1310) und dem zweiten optischen Teil (1350); und
ein Haptikreservoir (1303) zwischen dem ersten haptischen Teil (1341a) und dem zweiten haptischen Teil (1341b), wobei das Haptikreservoir (1303) mit der optischen Kammer (1305) in Fluidverbindung steht; und
eine feste Linse, die zum entfernbaren Koppeln mit der Basislinse konfiguriert ist, wobei die feste Linse Folgendes aufweist:
einen Linsenteil (1306) mit einer vorderen und einer hinteren Fläche, wobei der Linsenteil eine feste optische Leistung bietet, und
mehrere Laschen (1370), die sich vom Linsenteil (1306) radial nach außen erstrecken,
wobei, wenn die feste Linse entfernbar mit der Basislinse gekoppelt ist (1340) -
der Linsenteil (1306) mit dem ersten und zweiten optischen Teil (1310, 1350) ausgerichtet ist, und
jede Lasche (1370) innerhalb einer der mehreren Passstrukturen (1382) positioniert ist.

2. AIOL (1300) nach Anspruch 1, wobei die vordere Fläche der festen Linse hinter einer vordersten Kante der vorderen Basislinsenkomponente (1340a) positioniert ist, wenn die feste Linse mit der Basislinse (1340) gekoppelt ist.

3. AIOL (1300) nach Anspruch 1, die ferner ein Loch (1371) durch eine der mehreren Laschen (1370) umfasst, wobei das Loch (1371) zum Aufnehmen eines Teils eines Werkzeugs zum Manipulieren der festen Linse in Bezug auf die Basislinse (1340) konfiguriert ist.

4. AIOL (1300) nach Anspruch 1, wobei:
die vordere Basislinsenkomponente (1340a) einen ersten ringförmigen Passteil aufweist, der den ersten optischen Teil (1310) umgibt;
die hintere Basislinsenkomponente (1340b) einen zweiten ringförmigen Passteil aufweist, der den zweiten optischen Teil (1350) umgibt; und
der erste ringförmige Passteil mehrere Vorsprünge (1397) aufweist, die zum Verpaaren mit dem zweiten ringförmigen Passteil konfiguriert sind;
wobei Umfangsspalte zwischen den mehreren Vorsprüngen (1397) zumindest teilweise Fluidströmungswege (1349) zwischen dem Haptikreservoir (1303) und der optischen Kammer (1305) definieren.

5. AIOL (1300) nach Anspruch 1, wobei:
die vordere Basislinsenkomponente (1340a) einen ersten ringförmigen Passteil aufweist, der den ersten optischen Teil (1310) umgibt;
die hintere Basislinsenkomponente (1340b) einen zweiten ringförmigen Passteil umfasst, der den zweiten optischen Teil (1350) umgibt; und
der zweite ringförmige Passteil mehrere Vorsprünge (1397) umfasst, die zum Verpaaren mit dem ersten ringförmigen Passteil konfiguriert sind;
wobei Umfangsspalte zwischen den mehreren Vorsprüngen (1397) zumindest teilweise Fluidströmungswege (1349) zwischen dem Haptikreservoir (1303) und der optischen Kammer (1305) definieren.

6. AIOL (1300) nach Anspruch 1, die ferner eine oder mehrere Vertiefungen (1383) in einer vordersten Fläche der vorderen Basislinsenkomponente (1340a) aufweist.

7. AIOL (1300) nach Anspruch 6, wobei jedes der Durchflussmerkmale (1381) in Umfangsrichtung mit einer der ein oder mehreren Vertiefungen (1383) ausgerichtet ist.

8. AIOL (1300) nach Anspruch 1, die ferner eine Fluidkammer (1341) zwischen der festen Linse und dem ersten optischen Teil (1310) der vorderen Basislinsenkomponente (1340a) umfasst, wenn die feste Linse mit der Basislinse (1340) gekoppelt ist, wobei Umfangsspalte zwischen den Laschen (1370) der festen Linse das Strömen von Fluid um die feste Linse herum in die und aus der Fluidkammer (1341) zulassen.

9. AIOL (1300) nach Anspruch 1, die ferner einen Kanal (1391) in einer Außenwand der Basislinse umfasst, wobei sich der Kanal (1391) entlang des gesamten Umfangs der Basislinse (1340) erstreckt.

10. AIOL (1300) nach Anspruch 9, wobei der Kanal (1391) an einer Naht (1395) zwischen der vorderen Basislinsenkomponente (1340a) und der hinteren Basislinsenkomponente (1340b) positioniert ist.

11. AIOL (1300) nach Anspruch 1, wobei die mehreren Passstrukturen (1382) sich in Umfangsrichtung erstreckende Hohlräume umfassen, die zum Aufnehmen der mehreren Laschen (1370) konfiguriert sind.

12. AIOL (1300) nach Anspruch 1, die ferner mehrere Aufnahmestrukturen (1178) umfasst, die zum Aufnehmen der und lösbaren Verpaaren mit den mehreren Laschen (1370) der festen Linse konfiguriert sind.

13. AIOL (1300) nach Anspruch 12, wobei jede der mehreren Passstrukturen (1382) einen Hohlraum umfasst, der sich in Umfangsrichtung von einer der mehreren Aufnahmestrukturen (1178) erstreckt.

14. AIOL (1300) nach Anspruch 12, wobei-
wenn die feste Linse mit der Basislinse (1340) gekoppelt ist, die mehreren Laschen (1370) in den mehreren Aufnahmestrukturen (1178) aufgenommen sind; und
nach einer Drehung der festen Linse relativ zur Basislinse (1370) die mehreren Laschen (1370) in den mehreren Passstrukturen (1382) aufgenommen werden.

## Revendications

1. Lentille intraoculaire accommodative (AIOL) (1300) comprenant :
une lentille de base (1340) comportant
un composant de lentille de base antérieur (1340a) doté d'une première partie optique (1310) et d'une première partie haptique (1341a) qui entoure, au moins en partie, la première partie optique ;
un axe optique ;
un composant de lentille de base postérieur (1340b) couplé au composant de lentille de base antérieur (1340a) et doté d'une seconde partie optique (1350) alignée sur la première partie optique le long de l'axe optique ainsi que d'une seconde partie haptique (1341b) qui, au moins en partie, entoure la seconde partie optique et qui est alignée sur la première partie haptique dans une direction parallèle à l'axe optique ;
une pluralité de structures d'association (1382) formée dans l'un ou dans les deux composants du composant de lentille de base antérieur (1340a) et du composant de lentille de base postérieur (1340b), la pluralité de structures d'association (1382) s'ouvrant vers l'axe optique ;
une pluralité d'éléments d'écoulement de fluide (1381) formée par le composant de lentille de base antérieur (1340a) et par le composant de lentille de base postérieur (1340b), dans laquelle chaque élément d'écoulement de fluide (1381) sépare des structures individuelles des structures d'association (1382) ;
une chambre optique (1305) située entre la première partie optique (1310) et la seconde partie optique (1350) ; et
un réservoir haptique (1303) situé entre la première partie haptique (1341a) et la seconde partie haptique (1341b), le réservoir haptique (1303) étant en communication fluidique avec la chambre optique (1305) ; et
une lentille fixe configurée pour être couplée de manière amovible avec la lentille de base, la lentille fixe comportant une partie de lentille (1306) présentant une face antérieure et une face postérieure, la partie de lentille fournissant une puissance optique fixe, et
une pluralité de languettes (1370) s'étendant radialement vers l'extérieur à partir de la partie de lentille (1306),
dans laquelle, lorsque la lentille fixe est couplée de manière amovible à la lentille de base (1340) :
la partie de lentille (1306) est alignée sur la première et la seconde parties optiques (1310, 1350), et
chaque languette (1370) est logée à l'intérieur d'une structure de la pluralité de structures d'association (1382).

2. AIOL (1300) selon la revendication 1, dans laquelle la face antérieure de la lentille fixe est située derrière un bord le plus antérieur du composant de lentille de base antérieur (1340a) lorsque la lentille fixe est couplée à la lentille de base (1340).

3. AIOL (1300) selon la revendication 1, comprenant en outre un orifice (1371) pratiqué à travers une languette de la pluralité de languettes (1370), l'orifice (1371) étant configuré pour recevoir une partie d'un outil utilisé pour manipuler la lentille fixe par rapport à la lentille de base (1340).

4. AIOL (1300) selon la revendication 1, dans laquelle :
le composant de lentille de base antérieur (1340a) comprend une première partie d'association annulaire qui entoure la première partie optique (1310) ;
le composant de lentille de base postérieur (1340b) comprend une seconde partie d'association annulaire qui entoure la seconde partie optique (1350) ; et
la première partie d'association annulaire comprend une pluralité de saillies (1397) configurée pour s'associer à la seconde partie d'association annulaire ;
dans laquelle des espaces circonférentiels présents entre la pluralité de saillies (1397), au moins en partie, définissent des trajets d'écoulement de fluide (1349) entre le réservoir haptique (1303) et la chambre optique (1305).

5. AIOL (1300) selon la revendication 1, dans laquelle :
le composant de lentille de base antérieur (1340a) comprend une première partie d'association annulaire qui entoure la première partie optique (1310) ;
le composant de lentille de base postérieur (1340a) comprend une seconde partie d'association annulaire qui entoure la seconde partie optique (1350) ; et
la seconde partie d'association annulaire comprend une pluralité de saillies (1397) configurée pour s'associer à la première partie d'association annulaire ;
dans laquelle des espaces circonférentiels présents entre la pluralité de saillies (1397), au moins en partie, définissent des trajets d'écoulement de fluide (1349) entre le réservoir haptique (1303) et la chambre optique (1305).

6. AIOL (1300) selon la revendication 1, comprenant en outre une ou plusieurs indentations (1383) situées dans une surface la plus antérieure du composant de lentille de base antérieur (1340a).

7. AIOL (1300) selon la revendication 6, dans laquelle chacun des éléments d'écoulement de fluide (1381) est circonférentiellement aligné sur l'une de ladite indentation ou desdites plusieurs indentations (1383).

8. AIOL (1300) selon la revendication 1, comprenant en outre une chambre de fluide (1341) située entre la lentille fixe et la première partie optique (1310) du composant de lentille de base antérieur (1340a) lorsque la lentille fixe est couplée à la lentille de base (1340), dans laquelle des espaces circonférentiels présents entre les languettes (1370) de la lentille fixe permettent au fluide de circuler autour de la lentille fixe pour entrer dans la chambre de fluide (1341) et pour en sortir .

9. AIOL (1300) selon la revendication 1, comprenant en outre un canal (1391) situé dans une paroi externe de la lentille de base, le canal (1391) s'étendant le long de tout un paramètre de la lentille de base (1340).

10. AIOL (1300) selon la revendication 9, dans laquelle le canal (1391) est situé à hauteur d'un joint (1395) entre le composant de lentille de base antérieur (1340a) et le composant de lentille de base postérieur (1340b).

11. AIOL (1300) selon la revendication 1, dans laquelle la pluralité de structures d'association (1382) comprend des cavités s'étendant circonférentiellement qui sont configurées pour recevoir la pluralité des languettes (1370).

12. AIOL (1300) selon la revendication 1, comprenant en outre une pluralité de structures de réception (1178) configurée pour recevoir la pluralité de languettes (1370) de la lentille fixe et pour s'y associer de manière amovible.

13. AIOL (1300) selon la revendication 12, dans laquelle chaque structure de la pluralité de structures d'association (1382) comprend une cavité s'étendant circonférentiellement à partir d'une structure de la pluralité de structures de réception (1178).

14. AIOL (1300) selon la revendication 12, dans laquelle :
lorsque la lentille fixe est couplée à la lentille de base (1340), la pluralité de languettes (1370) vient se loger à l'intérieur de la pluralité des structures de réception (1178) ; et
suite à une rotation de la lentille fixe par rapport à la lentille de base (1370), la pluralité de languettes (1370) vient se loger à l'intérieur de la pluralité des structures d'association (1382).
